# EUROPEAN PATENT APPLICATION

(11) **EP 4 296 279 A1**
(43) Date of publication of application: **27.12.2023**
(21) Application number: 22180706.8
(22) Date of filing: 23.06.2022
(51) Int. Cl.: C07K 16/18, G01N 33/68, A61P 9/00, A61P 25/02

(54) **ANTI-TRANSTHYRETIN (TTR) BINDING PROTEINS AND USES THEREOF**

(71) Applicant: Fraunhofer-Gesellschaft zur Förderung der angewandten Forschung e.V., 80686 München (DE)
(72) Inventor: Köppen, Janett, 06110 Halle (Saale) (DE); Schiling, Stephan, 06130 Halle (Saale) (DE); Schulze, Anja, 06184 Kabelsketal (DE); Rahfeld, Jens-Ulrich, 06317 Seegebiet (DE); Wermann, Michael, 06118 Halle (Saale) (DE); Kleinschmidt, Martin, 06114 Halle (Saale) (DE); Cynis, Dr. Holger, 06120 Halle (DE)
(74) Representative: Hoffmann Eitle

(57) **Abstract**

Transthyretin amyloidosis is characterized by progressive deposition of the plasma protein transthyretin in the myocardium, peripheral nerves and/or other tissues, which can ultimately lead to congestive heart failure, polyneuropathy and death. The present invention provides binding proteins that can specifically bind to an isoaspartate residue in a transthyretin protein as well as their therapeutic, diagnostic and prognostic uses.

## Description

### Technical field

The present invention can be included in the field of medicine. Specifically, the present invention provides binding proteins that can specifically bind to an isoaspartate (isoD) residue in a transthyretin (TTR) protein as well as their use in the preventive and therapeutic treatment of transthyretin amyloidosis (ATTR). Further, the present invention provides the use of the binding protein in prognostic and/or diagnostic methods.

### Background art

Systemic amyloidoses represent a group of rare diseases that are often underdiagnosed due to their multifaceted symptomatic picture. In these multisystem diseases, misfolded fibrillar proteins are deposited extracellularly in various organs resulting in a successive loss of function and integrity (Nevone et al., 2020. Front Pharmacol. 11:1024).

Transthyretin amyloidosis (ATTR) is characterized by progressive deposition of the plasma protein transthyretin (TTR) in the myocardium, peripheral nerves and/or other tissues, which can ultimately lead to congestive heart failure, polyneuropathy and death. Usually patients die 4 to 12 years after diagnosis (Grogan et al., 2016. J Am Coll Cardiol. 68(10):1014-20; Ando et al., 2013. Orphanet J Rare Dis. 8:31).

There are two forms of ATTR, wild-type ATTR (ATTRwt) and hereditary ATTR (hATTR). ATTRwt is an often-underdiagnosed cause of heart failure, which mainly affects older individuals. In contrast, hATTR is an autosomal dominant hereditary disease caused by a mutation in the TTR gene. To date, over 130 different mutations are known, with varying age at disease onset and clinical presentation (Thomas et al., 2019. Neurodegener Dis Manag. 9(6):289-299). Some mutations predominantly cause polyneuropathy, whilst other mutations are cardiomyopathic or cause mixed phenotypes. The most prevalent TTR mutation worldwide is the Val30Met mutation, which typically results in peripheral neuropathy. The second most common hereditary mutation is Val122lle. Here, organ involvement may be limited to the heart (Adams et al., 2021. J Neurol. 268(6):2109-2122).

TTR is a 55 kDa homotetrameric protein that binds and transports thyroxine and holo-retinol binding protein in the cerebrospinal fluid and plasma (Buxbaum & Reixach, 2009. Cell Mol Life Sci. 66(19):3095-101). TTR is primarily produced in the liver. About 5 % of the total protein originates from the choroid plexus and retinal pigment epithelium (Gertz et al., 2015. J Am Coll Cardiol. 66(21):2451-2466). Factors such as low pH, elevated temperature or the presence of a mutation in the TTR gene lead to increased instability of the homotetrameric complex. They favor faster dissociation and the release of monomers, which in turn favors an aggregation process and formation of amyloid fibrils (Hurshman et al., 2004. Biochemistry. 43(23):7365-81). Recently, Schmidt *et al.* solved the first cryo-EM structure of a human ATTR-V30M fibril (Schmidt et al., 2019. Nat Commun. 10(1):5008). They showed that fibril formation involves an almost complete unfolding of the native conformation in order to allow the polypeptide chain to rearrange into a significantly different conformation. Moreover, the structures provide valuable insights into protein regions of the fibril, which might be prone to protein-protein interactions.

Therapeutic approaches for the treatment of ATTR include liver transplantation (Holmgren et al., 1993. Lancet. 341(8853):1113-6), gene silencing by siRNAs (patisiran, see Adams et al., 2018. NEngl J Med. 379(1):11-21) or antisense oligonucleotides (inotersen, Benson et al., 2018. N Engl J Med. 379(1):22-31) and stabilisation of the TTR complex in its naturally folded physiological form (tafamidis, see Maurer et al., 2018. N Engl J Med. 379(11): 1007-1016; diflunisal, see Lohrmann et al., 2020. J Card Fail. 26(9):753-759; epigallocatechin-3-gallate, see Ferreira et al., 2012. PLoS One. 7(1):e29933). These therapies are designed to prevent misfolding/unfolding, aggregation and deposition in the tissue.

Although these developments represent effective approaches to attenuate the progression of amyloid deposition, alternative measures are needed that can clear pre-existing deposits of TTR protein in the tissues of afflicted patients. Passive immunotherapy involving the administration of monoclonal antibodies (mAbs) directed against unnaturally folded, dissociated monomers and non-native oligomers or amyloid deposits to target them for degradation via Fc-mediated phagocytosis might represent a viable approach.

To our knowledge, all mAbs developed so far target a cryptic epitope on the surface of amyloidogenic TTR, which becomes exposed during the pathogenic formation of β-sheet fibrils and is thus unique to non-native TTR. Of these, the mAb PRX004 (Higaki et al., 2016. Amyloid. 23(2):86-97) has already passed phase 1 of clinical trials. The trials were, however, terminated prematurely due to the impact of the COVID-19 pandemic. Michalon *et al.* were able to develop NI006, another mAb for use in ATTR cardiomyopathy. Clinical trials involving the administration of NI006 are being planned (Michalon et al. 2021. Nat Commun. 12(1):3142).

There remains a need for effective therapeutics that can treat and/or prevent ATTR.

### Figures

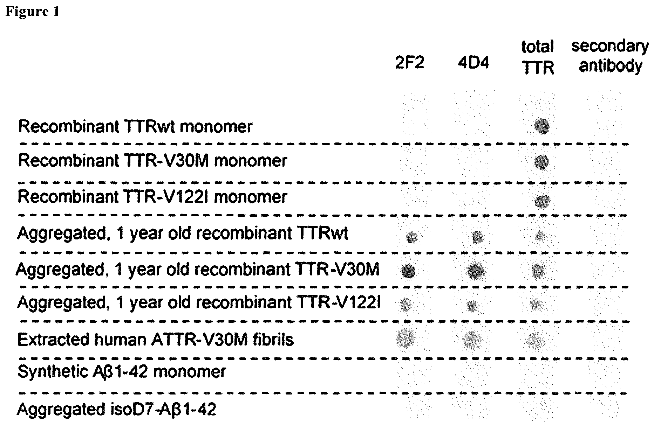

**Figure 1****: Specificity of the TTR mAbs 2F2 and 4D4, characterized by dot blot analysis.** The two mAbs specific for isoD-modified TTR (i.e., 2F2 and 4D4) and a polyclonal total TTR antibody were compared. The mAbs bind to aged amyloid aggregates of recombinant TTR protein and extracted fibrils. No binding was detected with native recombinant TTR monomers and aggregated Aβ1-42 (verified isoD content). Detection was carried out based on alkaline phosphatase activity. The data demonstrates the high specificity of the isoD-specific mAbs for aged, post-translationally modified TTR.

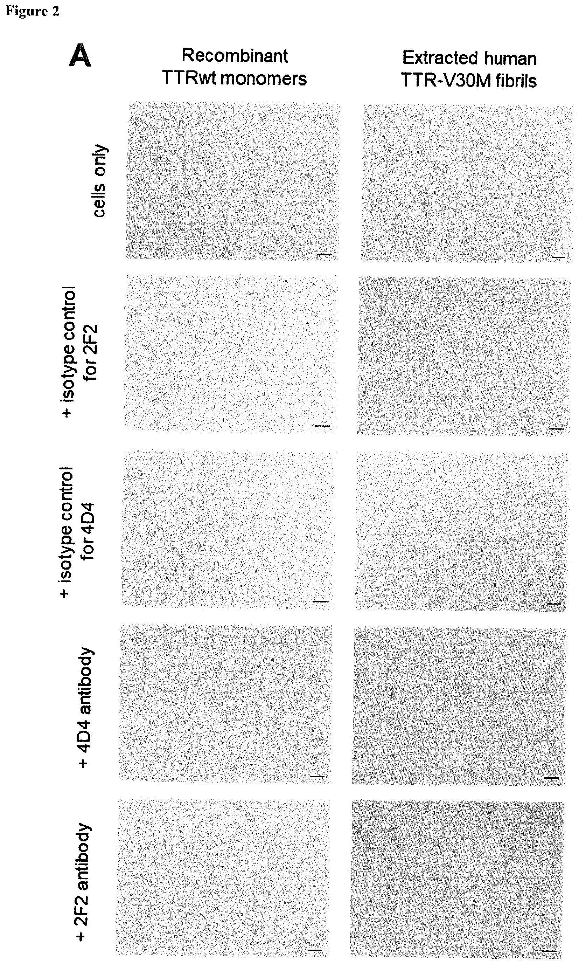

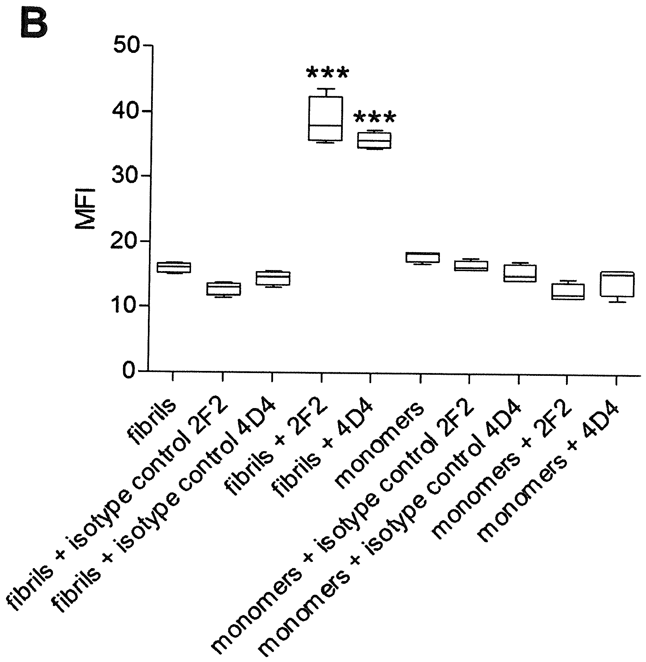

**Figure 2****: 2F2/4D4 mAb-dependent phagocytosis.** Recombinant TTRwt (wild-type TTR) proteins or extracted human ATTR-V30M fibrils were covalently labelled with the pH-sensitive fluorescent dye pHrodo Red, which shows enhanced fluorescence upon exposure to lowered pH in endocytic vesicles. (A) Overlays of bright field and fluorescence images of THP-1 cells reveal enhanced uptake of aggregated pHrodo-labelled ATTR-V30M fibrils in presence of isoD38-TTR-specific antibodies 2F2 and 4D4. Scale = 50 µm. (B) Engulfment of pHrodo-labelled fibrils was measured by relative mean fluorescence intensity (MFI) of THP-1 cells. Antibody-dependent phagocytosis was specific for pHrodo-ATTR-V30M fibrils that were treated with 2F2 or 4D4 antibody, but both isotype control antibodies failed to induce phagocytosis. 2F2 and 4D4 had no effect on the uptake of native recombinant TTRwt monomers. Data are represented by a boxplot with 1.5 IQR (interquartile range). Statistical analysis was done by a one-way ANOVA, Tukey post-hoc test (^{∗∗∗} = p < 0.001).

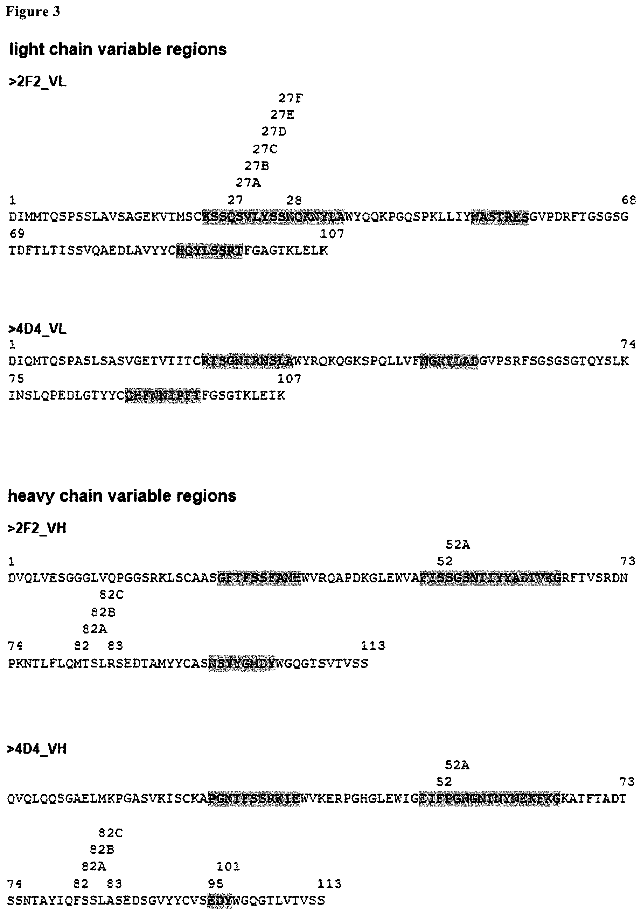

**Figure 3****: Kabat numbering scheme as applied to the VH and VL fragments of 2F2 and 4D4.** SEQ ID NOs: 17, 18, 27 and 28 are depicted in this figure.

### Summary of the invention

Recent progress in the treatment of other amyloidoses might provide guidance for the development of effective therapeutics for the treatment of ATTR. Donanemab, a mAb currently in phase 3 clinical trials for the treatment of Alzheimer's disease (AD), highlights the potential of tailored therapeutic approaches by targeting the pyroglutamate form of Aβ, a non-physiological post-translational modification (PTM) (Mintun et al., 2021. N Engl J Med. 384(18):1691-1704). In comparison, little is known about PTMs in TTR which may impact the development of ATTR.

Based on the comprehensive analysis of posttranslational modifications of Aβ and their function, some of these might be also deduced for TTR in ATTR. One of these modifications is the formation of isoaspartate (isoD), which results either from hydrolysis from L-aspartatyl or from deamidation of L-asparaginyl residues (Shimizu et al., 2000. Arch Biochem Biophys. 381(2):225-34), Isomerisation occurs spontaneously via an L-succinimidyl intermediate to L-isoaspartate or L-aspartate in a 3:1 ratio. This modification determines the half-life of a protein and significantly alters its structure by introducing an additional methylene group into the backbone of the protein (Robinson & Robinson, 2001. Proc Natl Acad Sci U S A. 98(3):944-9; Aswad et al., 2000. JPharm Biomed Anal. 21(6):1129-36; Geiger & Clarke, 1987. J Biol Chem. 262(2):785-94). This has a potential impact on the solubility, conformation and function of the protein.

Here, we provide evidence of isoaspartate formation at position 38 of the TTR protein and the presence of isoD38-TTR in amyloid deposits. We have developed two monoclonal antibodies 2F2 and 4D4, which specifically detect this modification in amyloid deposits in human pathological tissue. Native TTR protein is not bound by 2F2 and 4D4. Both antibodies promote cellular uptake of human isoD38-modified TTR fibrils into THP-1 cells by phagocytosis. Hence, we propose that these antibodies and other binding proteins that target isoD-modified TTRs have diagnostic and therapeutic potential.

The present invention thus provides a binding protein that specifically binds to an isoD-modified TTR. A nucleic acid comprising a sequence that encodes the binding protein of the invention is also provided, as well as a host cell comprising the nucleic acid of the present invention.

The present invention also provides a pharmaceutical composition comprising the binding protein, nucleic acid or host cell of the present invention and a pharmaceutically acceptable carrier or diluent. The binding protein, nucleic acid, host cell or pharmaceutical composition of the present invention may be for use as a medicament. In particular, the binding protein, nucleic acid, host cell or pharmaceutical composition of the present invention may be for use in the treatment and/or prevention of ATTR.

The present invention also provides a diagnostic and/or prognostic method for detecting the presence of isoD-modified TTR in a sample. The method may be an *in vitro* or *in vivo* method. The method may involve contacting the sample with the binding protein of the present invention.

An *in vitro* method involves an isolated sample. For example, the present invention provides an *in vitro* diagnostic and/or prognostic method for detecting the presence of isoD-modified TTR in an isolated sample, the method comprising contacting the isolated sample with the binding protein of the present invention, and determining whether isoD-modified TTR is present in the isolated sample.

The present invention also provides the binding protein of the present invention for use in a method of diagnosis and/or prognosis *in vivo* of ATTR.

### Detailed description of the invention

### Definitions

The term "*affibody*" refers to a protein that is derived from the Z domain of protein A and that been engineered to bind to a specific target (see Frejd & Kim, 2017. Exp Mol Med. 49(3): e306).

The term "*animal*", as used in the present application, refers to any multicellular eukaryotic heterotroph which is not a human. In some embodiments, the animal is selected from a group consisting of cats, dogs, pigs, ferrets, rabbits, gerbils, hamsters, guinea pigs, horses, rats, mice, cows, sheep, goats, alpacas, camels, donkeys, llamas, yaks, giraffes, elephants, meerkats, lemurs, lions, tigers, kangaroos, koalas, bats, monkeys, chimpanzees, gorillas, bears, dugongs, manatees, seals and rhinoceroses.

The term "*antibody*" refers to a molecule comprising at least one immunoglobulin domain that binds to and is specific for a particular target. The term includes whole antibodies and any antigen binding portion (e.g., F(ab')2, Fab', Fab, and Fv). The term includes horse, human, murine, rabbit, goat, donkey, camelid (e.g., llama, camel or alpaca), or cartilaginous fish (e.g., shark) antibodies or any antibodies derived thereof.

A typical type of antibody comprises at least two heavy chains ("HC") and two light chains ("*LC*") interconnected by disulfide bonds.

Each "*heavy chain*" comprises a "*heavy chain variable domain*" (abbreviated herein as "*VH*") and a "*heavy chain constant domain*" (abbreviated herein as "*CH*")*.* The heavy chain constant domain typically comprises three constants domains, i.e., CH1, CH2, and CH3.

Each "*light chain*" comprises a "*light chain variable domain*" (abbreviated herein as "*VL*") and a "light chain constant domain" ("*CL*")*.* The light chain constant domain (CL) can be of the kappa type or of the lambda type. The VH and VL domains can be further subdivided into regions of hypervariability, termed "*complementarity determining regions*" ("*CDR*"), interspersed with regions that are more conserved, termed "*framework regions*" ("*FW*")*.*

Each VH and VL is composed of three CDRs and four FWs, arranged from amino-terminus to carboxy-terminus in the following order: FW1, CDR1, FW2, CDR2, FW3, CDR3, FW4. The present disclosure *inter alia* presents VH and VL sequences as well as the subsequences corresponding to CDR1, CDR2, and CDR3.

Accordingly, a person skilled in the art would understand that the sequences of FW1, FW2, FW3 and FW4 are equally disclosed. For a particular VH, FW1 is the subsequence between the N-terminus of the VH and the N-terminus of HCDR1, FW2 is the subsequence between the C-terminus of HCDR1 and the N-terminus of HCDR2, FW3 is the subsequence between the C-terminus of HCDR2 and the N-terminus of HCDR3, and FW4 is the subsequence between the C-terminus of HCDR3 and the C-terminus of the VH. Similarly, for a particular VL, FW1 is the subsequence between the N-terminus of the VL and the N-terminus of LCDR1, FW2 is the subsequence between the C-terminus of LCDR1 and the N-terminus of LCDR2. FW3 is the subsequence between the C-terminus of LCDR2 and the N-terminus of LCDR3, and FW4 is the subsequence between the C-terminus of LCDR3 and the C-terminus of the VL.

The variable domains of the heavy and light chains contain a region that interacts with a binding target, and this region interacting with a binding target is also referred to as an "*antigen-binding site*" or "*antigen binding site*" herein. The constant domains of the antibodies can mediate the binding of the antibody to host tissues or factors, including various cells of the immune system (e.g., effector cells) and the first component (C1q) of the classical complement system. Exemplary antibodies of the present disclosure include typical antibodies, but also bivalent fragments and variations thereof such as a F(ab')2.

As used herein, the term "*antibody*" also encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising two antigen binding sites.

An antibody can be of any the five major classes (isotypes) of immunoglobulins: IgA, IgD, IgE, IgG, and IgM, or subclasses thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well-known subunit structures and three-dimensional configurations. Antibodies can be naked or conjugated to other molecules such as therapeutic agents or diagnostic agents to form immunoconjugates.

The term "*anticalin*" refers to a protein that is derived from the lipocalin and that been engineered to bind to a specific target (see Skerra, 2008. FEBS J. 275(11):2677-83).

The term "*binding protein*" refers to a protein that can specifically bind a target. Non-limiting examples of binding proteins include antibody, anticalin, repebody, monobody, scFv, scFab, affibody, fynomer, DARPin, nanobody, chimeric antigen receptor, chimeric engulfment receptor and peptide aptamer. The binding protein is preferably an antibody.

The term "*designed ankyrin repeat proteins*" or "*DARPin*" refers to a protein that is derived from an ankyrin repeat that has been engineered to bind to a specific target (see Plückthun, 2015. Annu Rev Pharmacol Toxicol. 55:489-511).

As used herein, the term "*effective amount*" of an agent, e.g., a therapeutic agent such as an antibody, is that amount sufficient to effect beneficial or desired results, for example, clinical results, and, as such, an "*effective amount*" depends upon the context in which it is being applied. For example, in the context of administering a therapeutic agent that treats ATTR, an effective amount of an agent is, for example, an amount sufficient to reduce or decrease TTR deposits, as compared to the response obtained without administration of the agent. The term "*effective amount*" can be used interchangeably with "*effective dose*", "*therapeutically effective amount*", or "*therapeutically effective dose*"*.*

The term "*fynomer*" refers to a protein that is derived from the SH3 domain of human Fyn kinase that has been engineered to bind to a specific target (see Bertschinger et al., 2007. Protein Eng Des Sel. 20(2):57-68).

The terms "*identical*" or "*percent identity*", in the context of two or more polypeptide or nucleic acid molecule sequences, means two or more sequences or subsequences that are the same or have a specified percentage of amino acid residues or nucleotides that are the same over a specified region (e.g., at least 60%, 65%, 70%, 75%, 80%, 85%, 90%, 91%, 92%, 93%, 94%, 95%, 96%, 97%, 98%, 99%, or 100% identity), when compared and aligned for maximum correspondence over a comparison window, or designated region, as measured using methods known in the art, such as a sequence comparison algorithm, by manual alignment, or by visual inspection. For example, preferred algorithms suitable for determining percent sequence identity and sequence similarity are the BLAST and BLAST 2.0 algorithms, which are described in Altschul et al., 1977. Nucleic Acids Res. 25:3389 and Altschul et al., 1990. J Mol Biol. 215:403, respectively. In some embodiments, the percent identity is determined over the length of the entire reference sequence disclosed herein.

The terms "*individual*", "*patient*" or "*subject*" are used interchangeably in the present application to designate a human being and are not meant to be limiting in any way. The "*individual*", "*patient*" or "*subject*" can be of any age, sex and physical condition.

The terms "*isoaspartate-modified transthyretin*", "*isoAsp-modified transthyretin*", "*isoAsp-modified TTR*", "*isoD-modified transthyretin*" and "*isoD-modified TTR*" refer to a transthyretin protein wherein an aspartate residue has been post-translationally modified into an isoaspartate (preferably an L-isoaspartate). An exemplary isoD-modified TTR is isoD38 TTR wherein the aspartate at position 38 (with reference to the numbering of SEQ ID NO: 1) has been post-translationally modified into an L-isoaspartate. Thus, isoD38 TTR refers to a TTR protein comprising SEQ ID NO: 3.

SEQ ID NO: 1 is an exemplary TTR having the following amino acid sequence:

GPTGTGESKCPLMVKVLDAVRGSPAINVAVHVFRKAADDTWEPFASGKTSESGELHGLTTEE
EFVEGIYKVEIDTKSYWKALGISPFHEHAEVVFTANDSGPRRYTIAALLSPYSYSTTAVVTNPK
E

The term *"K_{D}"* refers to the dissociation constant. In a preferred embodiment, the K_{D} is determined by surface plasmon resonance or isothermal titration calorimetry. Preferably, the K_{D} is determined by surface plasmon resonance at 25 °C.

The term "*monobody*" refers to a protein that is derived from a fibronectin type III domain that has been engineered to bind to a specific target (see Koide et al., 2013. J Mol Biol. 415(2):393-405).

The term "*nanobody*" refers to a protein comprising the soluble single antigen-binding V-domain of a heavy chain antibody, preferably a camelid heavy chain antibody (see Bannas et al., 2017. Front Immunol. 8:1603).

The term "*peptide aptamer*" refers to a short, 5-20 amino acid residue sequence that can bind to a specific target. Peptide aptamers are typically inserted within a loop region of a stable protein scaffold (see Reverdatto et al., 2015. Curr Top Med Chem. 15(12):1082-101).

The term "*prevention*", as used in the present application, refers to a set of hygienic, pharmacological, surgical and/or physical means used to prevent the onset and/or development of a disease and/or symptoms. The term "*prevention*" encompasses prophylactic methods, since these are used to maintain the health of an animal or individual.

The term "*repebody*" refers to a protein that is derived from a leucine-rich repeat module and that been engineered to bind to a specific target (see Lee et al., 2012. PNAS. 109(9): 3299-3304).

The term "*single-chain antigen-binding fragment*" or "*scFab*" refers to a fusion protein comprising one variable and one constant domain of the light chain of an antibody attached to one variable and one constant domain of the heavy chain of an antibody, wherein the heavy and light chains are linked together through a short peptide.

The term "*single-chain variable fragment*" or "*scFv*" refers to a fusion protein comprising the variable domains of the heavy chain and light chain of an antibody linked to one another with a peptide linker.

The term "*specifically binds*", within the context of the present invention, is used to describe a binding protein (e.g., antibody) that binds to a isoD-modified TTR with a K_{D} which is at least 10 times less (e.g., 20, 30, 40, 50, 60, 70, 80, 90, 100, 110, 120, 130, 140, 150, 160, 170, 180, 190 or 200 times less) than the K_{D} when the binding protein binds to a TTR which does not contain an isoD residue. In some embodiments, the epitope to which the binding protein specifically binds to comprises an isoD residue (preferably an isoD residue at position 38).

The terms "*treatment*" and "*therapy*", as used in the present application, refer to a set of hygienic, pharmacological, surgical and/or physical means used with the intent to cure and/or alleviate a disease and/or symptoms with the goal of remediating the health problem. The terms "*treatment*" and "*therapy*" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

### Binding protein

In a first aspect, the present invention provides a binding protein that specifically binds to an isoD-modified TTR.

In some embodiments, the binding protein specifically binds to isoD38 TTR. In some embodiments, the K_{D} of the interaction between the binding protein and a polypeptide (e.g., a TTR) comprising SEQ ID NO: 3 is at least 10 times less (e.g., at least 20, 30, 40, 50, 60, 70, 80, 90, 100, 110, 120, 130, 140, 150, 160, 170, 180, 190, 200, 300, 350, 400, or 450 times less) than the K_{D} of the interaction between the binding protein and a polypeptide (e.g., a TTR) comprising SEQ ID NO: 2. In a preferred embodiment, the K_{D} of the interaction between the binding protein and a polypeptide (e.g., a TTR) comprising SEQ ID NO: 3 is at least 100 times less than the K_{D} of the interaction between the binding protein and a polypeptide (e.g., a TTR) comprising SEQ ID NO: 2. More preferably, the K_{D} of the interaction between the binding protein and a polypeptide (e.g., a TTR) comprising SEQ ID NO: 3 is at least 150, 160, 170, 180, 190 or 200 times less than the K_{D} of the interaction between the binding protein and a polypeptide (e.g., a TTR) comprising SEQ ID NO: 2.

In some embodiments, the K_{D} of the interaction between the binding protein and SEQ ID NO: 3 is at least 10 times less (e.g., at least 20, 30, 40, 50, 60, 70, 80, 90, 100, 110, 120, 130, 140, 150, 160, 170, 180, 190, 200, 300, 350, 400, or 450 times less) than the K_{D} of the interaction between the binding protein and SEQ ID NO: 2. In a preferred embodiment, the K_{D} of the interaction between the binding protein and SEQ ID NO: 3 is at least 100 times less than the K_{D} of the interaction between the binding protein and SEQ ID NO: 2. More preferably, the K_{D} of the interaction between the binding protein and SEQ ID NO: 3 is at least 150, 160, 170, 180, 190 or 200 times less than the K_{D} of the interaction between the binding protein and SEQ ID NO: 2.

Preferably, the K_{D} is determined by surface plasmon resonance or isothermal titration calorimetry. More preferably, the K_{D} is determined by surface plasmon resonance at 25 °C. In some embodiments, the K_{D} is determined using SEQ ID NO: 5 for SEQ ID NO: 3 (in other words, the K_{D} is determined using a peptide consisting of SEQ ID NO: 3 with a PEG-biotin moiety covalently bound to the N-terminal residue). In some embodiments, the K_{D} is determined using SEQ ID NO: 6 for SEQ ID NO: 2 (in other words, the K_{D} is determined using a peptide consisting of SEQ ID NO: 2 with a PEG-biotin moiety covalently bound to the N-terminal residue).

In some embodiments, the K_{D} of the interaction between the binding protein and SEQ ID NO: 5 is at least 10 times less (e.g., at least 20, 30, 40, 50, 60, 70, 80, 90, 100, 110, 120, 130, 140, 150, 160, 170, 180, 190, 200, 300, 350, 400, or 450 times less) than the K_{D} of the interaction between the binding protein and SEQ ID NO: 6. In a preferred embodiment, the K_{D} of the interaction between the binding protein and SEQ ID NO: 5 is at least 100 times less than the K_{D} of the interaction between the binding protein and SEQ ID NO: 6. More preferably, the K_{D} of the interaction between the binding protein and SEQ ID NO: 5 is at least 150, 160, 170, 180, 190 or 200 times less than the K_{D} of the interaction between the binding protein and SEQ ID NO: 6.
SEQ ID NO: 2 is TTR (35-43) and has the following sequence:
   KAADDTWEP
SEQ ID NO: 3 is isoD38 TTR (35-43) and has the following sequence:
   KAAXDTWEP,
   wherein X is L-isoAsp.
SEQ ID NO: 5 is isoD38 TTR (35-43)PEG-Biotin and has the following sequence:
   KAAXDTWEP,
   wherein X is L-isoAsp, the N-terminal residue is modified with a PEG-Biotin moiety and the C-terminal residue is amidated.

In some embodiments, SEQ ID NO: 5 is a modified peptide according to Formula (I): 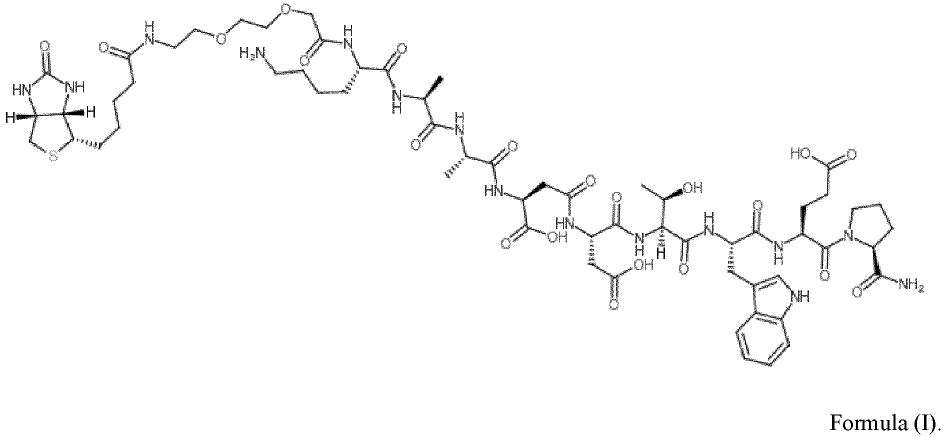 SEQ ID NO: 6 is TTR (35-43)PEG-Biotin and has the following sequence:
KAADDTWEP,
wherein the N-terminal residue is modified with a PEG-Biotin moiety and the C-terminal residue is amidated.

In some embodiments, SEQ ID NO: 6 is a modified peptide according to Formula (II):

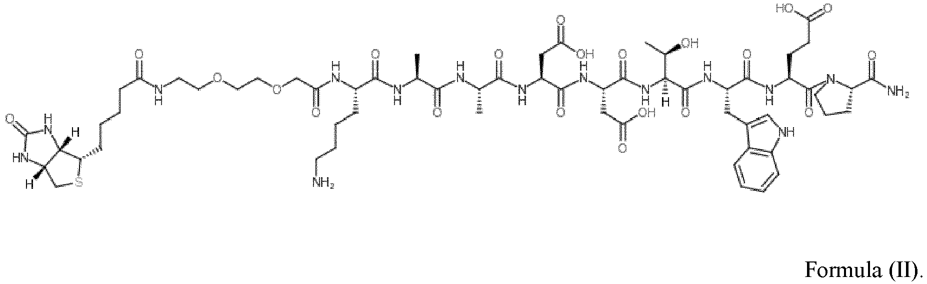

In some embodiments, the K_{D} of the interaction between the binding protein and a modified peptide according to Formula (I) is at least 10 times less (e.g., at least 20, 30, 40, 50, 60, 70, 80, 90, 100, 110, 120, 130, 140, 150, 160, 170, 180, 190, 200, 300, 350, 400, or 450 times less) than the K_{D} of the interaction between the binding protein and a modified peptide according to Formula (II). In a preferred embodiment, the K_{D} of the interaction between the binding protein and a modified peptide according to Formula (I) is at least 100 times less than the K_{D} of the interaction between the binding protein and a modified peptide according to Formula (II). More preferably, the K_{D} of the interaction between the binding protein and a modified peptide according to Formula (I) is at least 150, 160, 170, 180, 190 or 200 times less than the K_{D} of the interaction between the binding protein and a modified peptide according to Formula (II).

In a preferred embodiment, the K_{D} of the interaction of the binding protein and a polypeptide (e.g., a TTR) comprising SEQ ID NO: 3 at 25 °C is less than 200 nM. Preferably, the K_{D} is less than 150 nM. More preferably, the K_{D} is less than 10 nM. Most preferably, the K_{D} is less than 5 nM. Preferably, the K_{D} is determined by surface plasmon resonance or isothermal titration calorimetry. More preferably, the K_{D} is determined by surface plasmon resonance at 25 °C.

In a preferred embodiment, the K_{D} of the interaction of the binding protein and SEQ ID NO: 3 at 25 °C is less than 200 nM. Preferably, the K_{D} is less than 150 nM. More preferably, the K_{D} is less than 10 nM. Most preferably, the K_{D} is less than 5 nM. Preferably, the K_{D} is determined by surface plasmon resonance or isothermal titration calorimetry. More preferably, the K_{D} is determined by surface plasmon resonance at 25 °C. In some embodiments, the K_{D} is determined using SEQ ID NO: 5 for SEQ ID NO: 3 (in other words, the K_{D} is determined using a peptide consisting of SEQ ID NO: 3 with a PEG-biotin moiety covalently bound to the N-terminal residue).

In a preferred embodiment, the K_{D} of the interaction of the binding protein and SEQ ID NO: 5 at 25 °C is less than 200 nM. Preferably, the K_{D} is less than 150 nM. More preferably, the K_{D} is less than 10 nM. Most preferably, the K_{D} is less than 5 nM. Preferably, the K_{D} is determined by surface plasmon resonance or isothermal titration calorimetry. More preferably, the K_{D} is determined by surface plasmon resonance at 25 °C.

In a preferred embodiment, the K_{D} of the interaction of the binding protein and a modified peptide according to Formula (I) at 25 °C is less than 200 nM. Preferably, the K_{D} is less than 150 nM. More preferably, the K_{D} is less than 10 nM. Most preferably, the K_{D} is less than 5 nM. Preferably, the K_{D} is determined by surface plasmon resonance or isothermal titration calorimetry. More preferably, the K_{D} is determined by surface plasmon resonance at 25 °C.

In some embodiments, the binding protein comprises an Fc domain. An exemplary Fc domain may consist of the second and third constant domain (CH2-CH3) of a human IgG1 heavy chain. An Fc domain allows the binding protein to bind to Fc receptors and/or C1q thereby inducing antibody-dependent cell-mediated cytotoxicity, antibody-dependent cell-mediated phagocytosis and/or activation of the classical complement pathway. In an alternative embodiment, the binding protein is a chimeric antigen receptor (see, for example, Guedan et al., 2018 Mol Ther Methods Clin Dev. 12:145-156) or a chimeric engulfment receptor (see, for example, WO 2018/064076 A1).

In some embodiments, the binding protein is capable of inducing the phagocytosis of the isoD-modified TTR.

In some embodiments, the binding protein is an antibody, scFv, scFab, chimeric antigen receptor, or chimeric engulfment receptor.

In a preferred embodiment, the binding protein comprises:
(a) a heavy chain variable region (VH), wherein said VH comprises a HCDR3 polypeptide selected from NSYYGMDY (SEQ ID NO: 16) and EDY (SEQ ID NO: 26); and/or
(b) a light chain variable region (VL), wherein said VL comprises a LCDR3 polypeptide selected from HQYLSSRT (SEQ ID NO: 13) and QHFWNIPFT (SEQ ID NO: 23).

In a preferred embodiment, the binding protein comprises a light chain variable region (VL) and a heavy chain variable region (VH), wherein said VL comprises LCDR1, LCDR2 and LCDR3 polypeptides and VH comprises HCDR1, HCDR2 and HCDR3 polypeptides which are selected from the group consisting of:
(a) LCDR1 is KSSQSVLYSSNQKNYLA (SEQ ID NO: 11), LCDR2 is WASTRES (SEQ ID NO: 12), LCDR3 is HQYLSSRT (SEQ ID NO: 13), HCDR1 is GFTFSSFAMH (SEQ ID NO: 14), HCDR2 is FISSGSNTIYYADTVKG (SEQ ID NO: 15), and HCDR3 is NSYYGMDY (SEQ ID NO: 16); and
(b) LCDR1 is RTSGNIRNSLA (SEQ ID NO: 21), LCDR2 is NGKTLAD (SEQ ID NO: 22), LCDR3 is QHFWNIPFT (SEQ ID NO: 23), HCDR1 is GNTFSSRWIE (SEQ ID NO: 24), HCDR2 is EIFPGNGNTNYNEKFKG (SEQ ID NO: 25), and HCDR3 is EDY (SEQ ID NO: 26).

Variants of the CDRs set out in SEQ ID NOs: 11-26 are also envisioned by the present invention. Methods of identifying variant CDRs and, therefore, variant binding proteins are known in the art (see, for example, WO 2006/050491 A2).

An amino acid of a binding protein may be replaced by an amino acid having similar properties (based on size, polarity, hydrophobicity, and the like) to the amino acid to be replaced. In other words, the amino acid of a binding protein can be replaced with a different amino acid of the same class, where the amino acids may be classified as follows:
- aromatic: Phe, Tyr, Trp;
- apolar: Leu, Val, Ile, Ala, Met;
- aliphatic: Ala, Val, Leu, Ile;
- acidic: Asp, Glu;
- basic: His, Lys, Arg;
- polar: Gln, Asn, Ser, Thr, Tyr.

In certain embodiments, up to 50% (preferably up to 30%, more preferably up to 20%, even more preferably up to 10%) of the amino acid residues in one, two, three, four, five or six (preferably six) of the CDRs may be replaced according to the following table:

| **Amino acid to be replaced** | **Replacing amino acid** |
|---|---|
| Ala | Ser |
| Arg | Lys |
| Asn | Gln or His |
| Asp | Glu |
| Cys | Ser or Ala |
| Gln | Asn |
| Glu | Asp |
| Gly | Pro |
| His | Asn or Gln |
| Ile | Leu or Val |
| Leu | Ile or Val |
| Lys | Arg, Gln or Glu |
| Met | Leu or Ile |
| Phe | Met, Leu or Tyr |
| Pro | Gly |
| Ser | Thr |
| Thr | Ser |
| Trp | Tyr |
| Tyr | Trp or Phe |
| Val | Ile or Leu |

The amino acid of a binding protein may be replaced by a different amino acid that is present at the same position in a related binding protein (i.e., a binding protein that can bind to the same target). In the present application, 2F2 and 4D4 are related binding proteins.

In some embodiments, the present invention encompasses binding protein variants wherein 1, 2, 3, 4, 5, 6, 7, 8, 9, or 10 residues of the CDRs differ from the CDRs of 2F2 (i.e., SEQ ID NOs: 11-16) or 4D4 (i.e., SEQ ID NOs: 21-26).

In a preferred embodiment, the binding protein comprises:
(a) a heavy chain variable region (VH), wherein said VH comprises a HCDR3 polypeptide selected from NSYYGMDY (SEQ ID NO: 16) or a sequence that has at least 70% (preferably at least 80%) identity thereto and EDY (SEQ ID NO: 26) or a sequence that has at least 65% identity thereto; and/or
(b) a light chain variable region (VL), wherein said VL comprises a LCDR3 polypeptide selected from HQYLSSRT (SEQ ID NO: 13) or a sequence that has at least 70% (preferably at least 80%) identity thereto and QHFWNIPFT (SEQ ID NO: 23) or a sequence that has at least 70% (preferably at least 80%) identity thereto.

In a preferred embodiment, the binding protein comprises a light chain variable region (VL) and a heavy chain variable region (VH), wherein said VL comprises LCDR1, LCDR2 and LCDR3 polypeptides and VH comprises HCDR1, HCDR2 and HCDR3 polypeptides which are selected from the group consisting of:
(a) LCDR1 is KSSQSVLYSSNQKNYLA (SEQ ID NO: 11) or a sequence that has at least 70% (preferably at least 90%) identity thereto, LCDR2 is WASTRES (SEQ ID NO: 12) or a sequence that has at least 70% (preferably at least 80%) identity thereto, LCDR3 is HQYLSSRT (SEQ ID NO: 13) or a sequence that has at least 70% (preferably at least 80%) identity thereto, HCDR1 is GFTFSSFAMH (SEQ ID NO: 14) or a sequence that has at least 70% (preferably at least 90%) identity thereto, HCDR2 is FISSGSNTIYYADTVKG (SEQ ID NO: 15) or a sequence that has at least 70% (preferably at least 90%) identity thereto, and HCDR3 is NSYYGMDY (SEQ ID NO: 16) or a sequence that has at least 70% (preferably at least 80%) identity thereto; and
(b) LCDR1 is RTSGNIRNSLA (SEQ ID NO: 21) or a sequence that has at least 70% (preferably at least 90%) identity thereto, LCDR2 is NGKTLAD (SEQ ID NO: 22) or a sequence that has at least 70% (preferably at least 80%) identity thereto, LCDR3 is QHFWNIPFT (SEQ ID NO: 23) or a sequence that has at least 70% (preferably at least 80%) identity thereto, HCDR1 is GNTFSSRWIE (SEQ ID NO: 24) or a sequence that has at least 70% (preferably at least 90%) identity thereto, HCDR2 is EIFPGNGNTNYNEKFKG (SEQ ID NO: 25) or a sequence that has at least 70% (preferably at least 90%) identity thereto, and HCDR3 is EDY (SEQ ID NO: 26) or a sequence that has at least 65% identity thereto.

In a preferred embodiment, the binding protein comprises a VL and a VH, wherein the VL and VH are polypeptides selected from the group consisting of: (a) VL of SEQ ID NO: 17 and VH of SEQ ID NO: 18; and (b) VL of SEQ ID NO: 27 and VH of SEQ ID NO: 28.

In a preferred embodiment, the binding protein comprises a VL and a VH, wherein the VL and VH are polypeptides selected from the group consisting of: (a) VL of SEQ ID NO: 17 or a sequence that has at least 50% (e.g., at least 55%, 60%, 65%, 70%, 75%, 80% or 85%) identity thereto, and VH of SEQ ID NO: 18 or a sequence that has at least 50% (e.g., at least 55%, 60%, 65%, 70%, 75%, 80% or 85%) identity thereto; (b) VL of SEQ ID NO: 27 or a sequence that has at least 50% (e.g., at least 55%, 60%, 65%, 70%, 75%, 80% or 85%) identity thereto, and VH of SEQ ID NO: 28 or a sequence that has at least 50% (e.g., at least 55%, 60%, 65%, 70%, 75%, 80% or 85%) identity thereto; (c) VL of SEQ ID NO: 17 or a sequence that has at least 50% (e.g., at least 55%, 60%, 65%, 70%, 75%, 80% or 85%) identity thereto, and VH of SEQ ID NO: 28 or a sequence that has at least 50% identity thereto; and (d) VL of SEQ ID NO: 27 or a sequence that has at least 50% (e.g., at least 55%, 60%, 65%, 70%, 75%, 80% or 85%) identity thereto, and VH of SEQ ID NO: 18 or a sequence that has at least 50% (e.g., at least 55%, 60%, 65%, 70%, 75%, 80% or 85%) identity thereto.

In a preferred embodiment, the binding protein comprises a VL and a VH, wherein the VL and VH are polypeptides selected from the group consisting of: (a) VL of SEQ ID NO: 17 or a sequence that has at least 55% identity thereto, and VH of SEQ ID NO: 18 or a sequence that has at least 55% identity thereto; (b) VL of SEQ ID NO: 27 or a sequence that has at least 55% identity thereto, and VH of SEQ ID NO: 28 or a sequence that has at least 55% identity thereto; (c) VL of SEQ ID NO: 17 or a sequence that has at least 55% identity thereto, and VH of SEQ ID NO: 28 or a sequence that has at least 55% identity thereto; and (d) VL of SEQ ID NO: 27 or a sequence that has at least 55% identity thereto, and VH of SEQ ID NO: 18 or a sequence that has at least 55% identity thereto.

In a preferred embodiment, the binding protein comprises a VL and a VH, wherein the VL and VH are polypeptides selected from the group consisting of: (a) VL of SEQ ID NO: 17 or a sequence that has at least 60% identity thereto, and VH of SEQ ID NO: 18 or a sequence that has at least 60% identity thereto; (b) VL of SEQ ID NO: 27 or a sequence that has at least 60% identity thereto, and VH of SEQ ID NO: 28 or a sequence that has at least 60% identity thereto; (c) VL of SEQ ID NO: 17 or a sequence that has at least 60% identity thereto, and VH of SEQ ID NO: 28 or a sequence that has at least 60% identity thereto; and (d) VL of SEQ ID NO: 27 or a sequence that has at least 60% identity thereto, and VH of SEQ ID NO: 18 or a sequence that has at least 60% identity thereto.

In a preferred embodiment, the binding protein comprises a VL and a VH, wherein the VL and VH are polypeptides selected from the group consisting of: (a) VL of SEQ ID NO: 17 or a sequence that has at least 65% identity thereto, and VH of SEQ ID NO: 18 or a sequence that has at least 65% identity thereto; (b) VL of SEQ ID NO: 27 or a sequence that has at least 65% identity thereto, and VH of SEQ ID NO: 28 or a sequence that has at least 65% identity thereto; (c) VL of SEQ ID NO: 17 or a sequence that has at least 65% identity thereto, and VH of SEQ ID NO: 28 or a sequence that has at least 65% identity thereto; and (d) VL of SEQ ID NO: 27 or a sequence that has at least 65% identity thereto, and VH of SEQ ID NO: 18 or a sequence that has at least 65% identity thereto.

In a preferred embodiment, the binding protein comprises a VL and a VH, wherein the VL and VH are polypeptides selected from the group consisting of: (a) VL of SEQ ID NO: 17 or a sequence that has at least 70% identity thereto, and VH of SEQ ID NO: 18 or a sequence that has at least 70% identity thereto; (b) VL of SEQ ID NO: 27 or a sequence that has at least 70% identity thereto, and VH of SEQ ID NO: 28 or a sequence that has at least 70% identity thereto; (c) VL of SEQ ID NO: 17 or a sequence that has at least 70% identity thereto, and VH of SEQ ID NO: 28 or a sequence that has at least 70% identity thereto; and (d) VL of SEQ ID NO: 27 or a sequence that has at least 70% identity thereto, and VH of SEQ ID NO: 18 or a sequence that has at least 70% identity thereto.

In a preferred embodiment, the binding protein comprises a VL and a VH, wherein the VL and VH are polypeptides selected from the group consisting of: (a) VL of SEQ ID NO: 17 or a sequence that has at least 75% identity thereto, and VH of SEQ ID NO: 18 or a sequence that has at least 75% identity thereto; (b) VL of SEQ ID NO: 27 or a sequence that has at least 75% identity thereto, and VH of SEQ ID NO: 28 or a sequence that has at least 75% identity thereto; (c) VL of SEQ ID NO: 17 or a sequence that has at least 75% identity thereto, and VH of SEQ ID NO: 28 or a sequence that has at least 75% identity thereto; and (d) VL of SEQ ID NO: 27 or a sequence that has at least 75% identity thereto, and VH of SEQ ID NO: 18 or a sequence that has at least 75% identity thereto.

In a preferred embodiment, the binding protein comprises a VL and a VH, wherein the VL and VH are polypeptides selected from the group consisting of: (a) VL of SEQ ID NO: 17 or a sequence that has at least 80% identity thereto, and VH of SEQ ID NO: 18 or a sequence that has at least 80% identity thereto; (b) VL of SEQ ID NO: 27 or a sequence that has at least 80% identity thereto, and VH of SEQ ID NO: 28 or a sequence that has at least 80% identity thereto; (c) VL of SEQ ID NO: 17 or a sequence that has at least 80% identity thereto, and VH of SEQ ID NO: 28 or a sequence that has at least 80% identity thereto; and (d) VL of SEQ ID NO: 27 or a sequence that has at least 80% identity thereto, and VH of SEQ ID NO: 18 or a sequence that has at least 80% identity thereto.

In a preferred embodiment, the binding protein comprises a VL and a VH, wherein the VL and VH are polypeptides selected from the group consisting of: (a) VL of SEQ ID NO: 17 or a sequence that has at least 85% identity thereto, and VH of SEQ ID NO: 18 or a sequence that has at least 85% identity thereto; (b) VL of SEQ ID NO: 27 or a sequence that has at least 85% identity thereto, and VH of SEQ ID NO: 28 or a sequence that has at least 85% identity thereto; (c) VL of SEQ ID NO: 17 or a sequence that has at least 85% identity thereto, and VH of SEQ ID NO: 28 or a sequence that has at least 85% identity thereto; and (d) VL of SEQ ID NO: 27 or a sequence that has at least 85% identity thereto, and VH of SEQ ID NO: 18 or a sequence that has at least 85% identity thereto.
SEQ ID NO: 17 is the VL fragment present in 2F2 and has the following sequence: 
NIMMTQSPSSLAVSAGEKVTMSCKSSQSVLYSSNQKNYLAWYQQKPGQSPKLLIYWASTRES
GVPDRFTGSGSGTDFTLTISSVQAEDLAVYYCHQYLSSRTFGAGTKLELK
SEQ ID NO: 18 is the VH fragment present in 2F2 and has the following sequence: 
DVQLVESGGGLVQPGGSRKLSCAASGFTFSSFAMHWVRQAPDKGLEWVAFISSGSNTIYYAD
TVKGRFTVSRDNPKNTLFLQMTSLRSEDTAMYYCASNSYYGMDYWGQGTSVTVSS
SEQ ID NO: 27 is the VL fragment present in 4D4 and has the following sequence: 
DIQMTQSPASLSASVGETVTITCRTSGNIRNSLAWYRQKQGKSPQLLVFNGKTLADGVPSRFS
GSGSGTQYSLKINSLQPEDLGTYYCQHFWNIPFTFGSGTKLEIK
SEQ ID NO: 28 is the VH fragment present in 4D4 and has the following sequence:

QVQLQQSGAELMKPGASVKISCKAPGNTFSSRWIEWVKERPGHGLEWIGEIFPGNGNTNYNE
KFKGKATFTADTSSNTAYIQFSSLASEDSGVYYCVSEDYWGQGTLVTVSS

Preferably, the binding protein is an antibody.

Phage display and combinatorial methods for generating antibodies are known in the art (as described in, e.g., Ladner et al. U.S. Patent No.5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; and Barbas et al. (1991) PNAS 88:7978-7982).

In one embodiment, the antibody is a fully human antibody (e.g., an antibody made in a mouse which has been genetically engineered to produce an antibody from a human immunoglobulin sequence), or a non-human antibody, e.g., a rodent (mouse or rat), goat, primate (e.g., monkey), camel antibody. Preferably, the non-human antibody is a rodent (mouse or rat) antibody. Methods of producing rodent antibodies are known in the art.

Human monoclonal antibodies can be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, e.g., Wood et al. International Application WO 91/00906, Kucherlapati et al. PCT publication WO 91/10741; Lonberg et al. International Application WO 92/03918; Kay et al. International Application 92/03917; Lonberg, N. et al.1994 Nature 368:856-859; Green, L.L. et al.1994 Nature Genet. 7:13-21; Morrison, S.L. et al.1994 Proc. Natl. Acad. Sci. USA 81:6851-6855; Bruggeman et al. 1993 Year Immunol 7:33-40; Tuaillon et al.1993 PNAS 90:3720-3724; Bruggeman et al.1991 Eur J Immunol 21:1323-1326).

An antibody can be one in which the variable region, or a portion thereof, e.g., the CDRs, are generated in a non-human organism, e.g., a rat or mouse. Chimeric, CDR-grafted, and humanized antibodies are within the invention. Antibodies generated in a non-human organism, e.g., a rat or mouse, and then modified, e.g., in the variable framework or constant region, to decrease antigenicity in a human are within the invention.

Chimeric antibodies can be produced by recombinant DNA techniques known in the art (see Robinson et al., International Patent Publication PCT/US86/02269; Akira, et al., European Patent Application 184,187; Taniguchi, M., European Patent Application 171,496; Morrison et al., European Patent Application 173,494; Neuberger et al., International Application WO 86/01533; Cabilly et al. U.S. Patent No.4,816,567; Cabilly et al., European Patent Application 125,023; Better et al. (1988 Science 240:1041-1043); Liu et al. (1987) PNAS 84:3439-3443; Liu et al., 1987, J. Immunol.139:3521-3526; Sun et al. (1987) PNAS 84:214-218; Nishimura et al., 1987, Canc. Res.47:999-1005; Wood et al. (1985) Nature 314:446-449; and Shaw et al., 1988, J. Natl Cancer Inst.80:1553-1559).

A humanized or CDR-grafted antibody will have at least one or two but generally all three recipient CDRs (of heavy and or light immuoglobulin chains) replaced with a donor CDR. The antibody may be replaced with at least a portion of a non-human CDR or only some of the CDRs may be replaced with non-human CDRs. It is only necessary to replace the number of CDRs required for binding of the humanized antibody to isoD-modified TTR. Preferably, the donor will be a rodent antibody, e.g., a rat or mouse antibody, and the recipient will be a human framework or a human consensus framework. Typically, the immunoglobulin providing the CDRs is called the "*donor*" and the immunoglobulin providing the framework is called the "*acceptor*"*.* In one embodiment, the donor immunoglobulin is a non-human (e.g., rodent) immunoglobulin. The acceptor framework is a naturally-occurring (e.g., a human) framework or a consensus framework, or a sequence about 85% or higher, preferably 90%, 95%, 99% or higher identical thereto.

As used herein, the term "*consensus sequence*" refers to the sequence formed from the most frequently occurring amino acids (or nucleotides) in a family of related sequences (see e.g., Winnaker, From Genes to Clones (Verlagsgesellschaft, Weinheim, Germany 1987)). In a family of proteins, each position in the consensus sequence is occupied by the amino acid occurring most frequently at that position in the family. If two amino acids occur equally frequently, either can be included in the consensus sequence. A "*consensus framework*" refers to the framework region in the consensus immunoglobulin sequence.

An antibody can be humanized by methods known in the art (see e.g., Morrison, S. L., 1985, Science 229:1202-1207, by Oi et al., 1986, BioTechniques 4:214, and by Queen et al. US 5,585,089, US 5,693,761 and US 5,693,762).

Humanized or CDR-grafted antibodies can be produced by CDR-grafting or CDR substitution, wherein one, two, or all CDRs of an immunoglobulin chain can be replaced. See e.g., U.S. Patent 5,225,539; Jones et al.1986 Nature 321:552-525; Verhoeyan et al.1988 Science 239:1534; Beidler et al.1988 J. Immunol.141:4053-4060; Winter US 5,225,539. Winter describes a CDR-grafting method which may be used to prepare the humanized antibodies of the present invention (UK Patent Application GB 2188638A, filed on March 26, 1987; Winter US 5,225,539).

Also within the scope of the invention are humanized antibodies in which specific amino acids have been substituted, deleted or added. Criteria for selecting amino acids from the donor are described in US 5,585,089, e.g., columns 12-16 of US 5,585,089, e.g., columns 12-16 of US 5,585,089. Other techniques for humanizing antibodies are described in Padlan et al. EP 519596 A1, published on December 23, 1992.

In yet other embodiments, the antibody has a heavy chain constant region chosen from, e.g., the heavy chain constant regions of IgG1, IgG2, IgG3, IgG4, IgM, IgA1, IgA2, IgD, and IgE; particularly, chosen from, e.g., the (e.g., human) heavy chain constant regions of IgG1, IgG2, IgG3, and IgG4. In another embodiment, the antibody has a light chain constant region chosen from, e.g., the (e.g., human) light chain constant regions of kappa or lambda. The constant region can be altered, e.g., mutated, to modify the properties of the antibody (e.g., to increase or decrease one or more of: Fc receptor binding, antibody glycosylation, the number of cysteine residues, effector cell function, and/or complement function). In one embodiment the antibody has effector function and can fix complement. In other embodiments the antibody does not recruit effector cells or fix complement. In another embodiment, the antibody has reduced or no ability to bind an Fc receptor. For example, it is a isotype or subtype, fragment or other mutant, which does not support binding to an Fc receptor, e.g., it has a mutagenized or deleted Fc receptor binding region.

Methods for altering an antibody constant region are known in the art. Antibodies with altered function, e.g. altered affinity for an effector ligand, such as FcR on a cell, or the C1 component of complement can be produced by replacing at least one amino acid residue in the constant portion of the antibody with a different residue (see e.g., EP 388,151 A1, U.S. Pat. No. 5,624,821 and U.S. Pat. No.5,648,260). Similar type of alterations could be described which if applied to the murine, or other species immunoglobulin would reduce or eliminate these functions.

An antibody can be derivatized or linked to another functional molecule (e.g., another peptide or protein). As used herein, a "*derivatized*" antibody molecule is one that has been modified. Methods of derivatization include but are not limited to the addition of a fluorescent moiety, a radionucleotide, a toxin, an enzyme or an affinity ligand such as biotin. Accordingly, the antibody molecules of the invention are intended to include derivatized and otherwise modified forms of the antibodies described herein, including immunoadhesion molecules. For example, an antibody molecule can be functionally linked (by chemical coupling, genetic fusion, noncovalent association or otherwise) to one or more other molecular entities, such as another antibody (e.g., a bispecific antibody or a diabody), a detectable agent, a cytotoxic agent, a pharmaceutical agent, and/or a protein or peptide that can mediate association of the antibody or antibody portion with another molecule (such as a streptavidin core region or a polyhistidine tag).

One type of derivatized antibody molecule is produced by crosslinking two or more antibodies (of the same type or of different types, e.g., to create bispecific antibodies). Suitable crosslinkers include those that are heterobifunctional, having two distinctly reactive groups separated by an appropriate spacer (e.g., m-maleimidobenzoyl-N-hydroxysuccinimide ester) or homobifunctional (e.g., disuccinimidyl suberate). Such linkers are available from Pierce Chemical Company, Rockford, Ill.

An antibody molecule may be conjugated to another molecular entity, typically a label or a therapeutic (e.g., a cytotoxic or cytostatic) agent or moiety. Radioactive isotopes can be used in diagnostic or therapeutic applications. Such radioactive isotopes include, but are not limited to iodine (¹³¹I or ¹²⁵I), yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), indium (¹¹¹In), technetium (⁹⁹mTc), phosphorus (³²P), rhodium (¹⁸⁸Rh), sulfur (³⁵S) , carbon (¹⁴C), tritium (³H), chromium (⁵¹Cr), chlorine (³⁶Cl), cobalt (⁵⁷Co or ⁵⁸Co), iron (⁵⁹Fe), selenium (75Se), or gallium (67Ga). Radioisotopes useful as labels, e.g., for use in diagnostics, include iodine (¹³¹I or ¹²⁵I), indium (¹¹¹In), technetium (⁹⁹mTc), phosphorus (³²P), carbon (¹⁴C), and tritium (³H), or one or more of the therapeutic isotopes listed above.

The invention provides radiolabeled antibody molecules and methods of labeling the same. In one embodiment, a method of labeling an antibody molecule is disclosed. The method includes contacting an antibody molecule, with a chelating agent, to thereby produce a conjugated antibody. The conjugated antibody is radiolabeled with a radioisotope, e.g., 111Indium, 90Yttrium and 177Lutetium, to thereby produce a labeled antibody molecule.

The antibody molecule can be conjugated to a therapeutic agent. The antibody may be labeled. For example, the antibody may be labeled with a biotin molecule, an enzyme or a fluorophore.

The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("*Kabat*" numbering scheme), Al-Lazikani et al., (1997) JMB 273,927-948 ("*Chothia*" numbering scheme). As used herein, the CDRs defined according the "*Chothia*" number scheme are also sometimes referred to as "*hypervariable loops*"*.* In some embodiments, the amino acid sequence boundaries of the CDRs are determined using the Kabat numbering scheme. An exemplary application of the Kabat numbering scheme is provided in Figure 3.

In a preferred embodiment, the antibody or antigen-binding fragment thereof comprises two LCs and two HCs, wherein each LC and each HC are polypeptides selected from the group consisting of: (a) LC of SEQ ID NO: 31 and HC of SEQ ID NO: 32; (b) LC of SEQ ID NO: 33 and HC of SEQ ID NO: 34; (c) LC of SEQ ID NO: 31 and HC of SEQ ID NO: 34; and (d) LC of SEQ ID NO: 33 and HC of SEQ ID NO: 32.
SEQ ID NO: 31 is the LC present in 2F2 and has the following sequence: 
NIMMTQSPSSLAVSAGEKVTMSCKSSQSVLYSSNQKNYLAWYQQKPGQSPKLLIYWASTRES
GVPDRFTGSGSGTDFTLTISSVQAEDLAVYYCHQYLSSRTFGAGTKLELKRADAAPTVSIFPPS
SEQLTSGGASVVCFLNNFYPKDINVKWKIDGSERQNGVLNSWTDQDSKDSTYSMSSTLTLTK
DEYERHNSYTCEATHKTSTSPIVKSFNRNEC
SEQ ID NO: 32 is the HC present in 2F2 and has the following sequence: 
DVQLVESGGGLVQPGGSRKLSCAASGFTFSSFAMHWVRQAPDKGLEWVAFISSGSNTIYYAD
TVKGRFTVSRDNPKNTLFLQMTSLRSEDTAMYYCASNSYYGMDYWGQGTSVTVSSATTTAP
SVYPLVPGCSDTSGSSVTLGCLVKGYFPEPVTVKWNYGALSSGVRTVSSVLQSGFYSLSSLVT
VPSSTWPSQTVICNVAHPASKTELIKRIEPRIPKPSTPPGSSCPPGNILGGPSVFIFPPKPKDALMI
SLTPKVTCVVVDVSEDDPDVHVSWFVDNKEVHTAWTQPREAQYNSTFRVVSALPIQHQDW
MRGKEFKCKVNNKALPAPIERTISKPKGRAQTPQVYTIPPPREQMSKKKVSLTCLVTNFFSEAI
SVEWERNGELEQDYKNTPPILDSDGTYFLYSKLTVDTDSWLQGEIFTCSVVHEALHNHHTQK
NLSRSPGK
SEQ ID NO: 33 is the LC present in 4D4 and has the following sequence: 
DIQMTQSPASLSASVGETVTITCRTSGNIRNSLAWYRQKQGKSPQLLVFNGKTLADGVPSRFS
GSGSGTQYSLKINSLQPEDLGTYYCQHFWNIPFTFGSGTKLEIKRADAAPTVSIFPPSSEQLTSG
GASVVCFLNNFYPKDINVKWKIDGSERQNGVLNSWTDQDSKDSTYSMSSTLTLTKDEYERH
NSYTCEATHKTSTSPIVKSFNRNEC
SEQ ID NO: 34 is the HC present in 4D4 and has the following sequence:

QVQLQQSGAELMKPGASVKISCKAPGNTFSSRWIEWVKERPGHGLEWIGEIFPGNGNTNYNE
KFKGKATFTADTSSNTAYIQFSSLASEDSGVYYCVSEDYWGQGTLVTVSSAKTTAPSVYPLAP
VCGDTTGSSVTLGCLVKGYFPEPVTLTWNSGSLSSGVHTFPAVLQSDLYTLSSSVTVTSSTWP
SQSITCNVAHPASSTKVDKKIEPRGPTIKPCPPCKCPAPNLLGGPSVFIFPPKIKDVLMISLSPIVT
CVVVDVSEDDPDVQISWFVNNVEVHTAQTQTHREDYNSTLRVVSALPIQHQDWMSGKEFKC
KVNNKDLPAPIERTISKPKGSVRAPQVYVLPPPEEEMTKKQVTLTCMVTDFMPEDIYVEWTN
NGKTELNYKNTEPVLDSDGSYFMYSKLRVEKKNWVERNSYSCSVVHEGLHNHHTTKSFSRT
PGK

In a preferred embodiment, the antibody is a monoclonal antibody.

### Nucleic acid

In a second aspect, the present invention provides a nucleic acid comprising a sequence that encodes the binding protein of the present invention. In a preferred embodiment, the sequence is codon optimized to ensure high expression of the fusion protein. Methods for optimizing the codons of a genetic construct are known in the art (Quax et al., 2015. Mol Cell. 59(2): 149-61).

In a preferred embodiment, the nucleic acid is comprised in a vector. The vector may be a cloning vector, expression vector, transfer vector or viral vector.

In some embodiments, the nucleic acid is a modified mRNA molecule suitable for expressing the binding protein *in vivo.* The mRNA may be delivered using lipid nanoparticles (see Hou et al., 2021. Nat Rev Mater. 6(12):1078-1094).

In some embodiments, the nucleic acid comprises:
(a) SEQ ID NO: 19 and SEQ ID NO: 20;
(b) SEQ ID NO: 29 and SEQ ID NO: 30;
(c) SEQ ID NO: 29 and SEQ ID NO: 20; or
(d) SEQ ID NO: 19 and SEQ ID NO: 30.

SEQ ID NO: 19 is a nucleic acid sequence encoding the VL fragment of 2F2: 
AACATTATGATGACGCAGTCGCCATCATCTCTGGCTGTGTCTGCAGGAGAAAAGGTCACTATGAGCTGTAAGT
CCAGTCAGAGTGTTTTATACAGTTCAAATCAGAAGAATTACTTGGCCTGGTACCAGCAGAAACCAGGGCAGTC
TCCTAAACTGCTGATCTACTGGGCATCCACTAGGGAATCTGGTGTCCCTGATCGCTTCACAGGCAGTGGATCTG
GGACAGATTTTACTCTCACCATCAGCAGTGTACAAGCTGAAGACCTGGCAGTTTATTACTGTCATCAATACCTC
TCCTCGCGCACGTTCGGTGCTGGGACCAAGCTGGAGCTGAAG
SEQ ID NO: 20 is a nucleic acid sequence encoding the VH fragment of 2F2: 
GATGTGCAGCTGGTGGAGTCTGGGGGAGGCTTAGTGCAGCCTGGAGGGTCCCGGAAACTCTCCTGTGCAGCCT
CTGGATTCACTTTCAGTAGCTTTGCAATGCACTGGGTTCGTCAGGCTCCAGACAAGGGGCTGGAGTGGGTCGCA
TTCATTAGTAGTGGCAGTAATACCATCTACTATGCAGACACAGTGAAGGGCCGATTCACCGTCTCCAGAGACA
ATCCCAAGAACACCCTGTTCCTGCAAATGACCAGTCTAAGGTCTGAGGACACGGCCATGTATTACTGTGCAAG
TAATTCGTACTATGGTATGGACTACTGGGGTCAAGGAACCTCAGTCACCGTCTCCTCA
SEQ ID NO: 29 is a nucleic acid sequence encoding the VL fragment of 4D4: 
GACATCCAGATGACTCAGTCTCCAGCCTCCCTATCTGCATCTGTGGGAGAAACTGTCACCATCACATGTCGAAC
AAGTGGGAATATTCGCAATTCATTAGCATGGTATCGACAGAAACAGGGAAAATCCCCTCAGCTCCTGGTCTTT
AATGGCAAAACTTTAGCAGATGGTGTGCCATCAAGGTTCAGTGGCAGTGGATCAGGAACACAATATTCTCTCA
AGATCAACAGCCTGCAGCCTGAAGATTTAGGGACTTATTACTGTCAACATTTTTGGAATATTCCATTCACGTTC
GGCTCGGGGACAAAGTTGGAAATAAAA
SEQ ID NO: 30 is a nucleic acid sequence encoding the VH fragment of 4D4:

CAGGTTCAGCTGCAGCAGTCTGGAGCTGAGCTGATGAAGCCTGGGGCCTCAGTGAAGATATCCTGCAAGGCTC
CTGGCAACACATTCAGTAGCAGGTGGATAGAGTGGGTAAAGGAGAGGCCTGGACATGGCCTTGAGTGGATTG
GAGAGATTTTTCCTGGAAATGGTAATACTAACTACAATGAGAAGTTCAAGGGCAAGGCCACATTCACTGCAGA
TACATCCTCCAACACAGCCTATATACAATTCAGCAGCCTGGCATCTGAGGACTCTGGCGTCTATTACTGTGTAT
CTGAAGACTATTGGGGCCAAGGTACCCTGGTCACCGTCTCCTCA

In some embodiments, the nucleic acid comprises:
(a) SEQ ID NO: 35 and SEQ ID NO: 36;
(b) SEQ ID NO: 37 and SEQ ID NO: 38;
(c) SEQ ID NO: 35 and SEQ ID NO: 38; or
(d) SEQ ID NO: 37 and SEQ ID NO: 36.

SEQ ID NO: 35 is a nucleic acid sequence encoding the LC of 2F2: 
AACATTATGATGACGCAGTCGCCATCATCTCTGGCTGTGTCTGCAGGAGAAAAGGTCACTATGAGCTGTAAGT
CCAGTCAGAGTGTTTTATACAGTTCAAATCAGAAGAATTACTTGGCCTGGTACCAGCAGAAACCAGGGCAGTC
 
TCCTAAACTGCTGATCTACTGGGCATCCACTAGGGAATCTGGTGTCCCTGATCGCTTCACAGGCAGTGGATCTG
GGACAGATTTTACTCTCACCATCAGCAGTGTACAAGCTGAAGACCTGGCAGTTTATTACTGTCATCAATACCTC
TCCTCGCGCACGTTCGGTGCTGGGACCAAGCTGGAGCTGAAGCGGGCTGATGCTGCACCAACTGTATCCATCTT
CCCACCATCCAGTGAGCAGTTAACATCTGGAGGTGCCTCAGTCGTGTGCTTCTTGAACAACTTCTACCCCAAAG
ACATCAATGTCAAGTGGAAGATTGATGGCAGTGAACGACAAAATGGCGTCCTGAACAGTTGGACTGATCAGGA
CAGCAAAGACAGCACCTACAGCATGAGCAGCACCCTCACGTTGACCAAGGACGAGTATGAACGACATAACAG
CTATACCTGTGAGGCCACTCACAAGACATCAACTTCACCCATTGTCAAGAGCTTCAACAGGAATGAGTGTTAG
SEQ ID NO: 36 is a nucleic acid sequence encoding the HC of 2F2: 
GATGTGCAGCTGGTGGAGTCTGGGGGAGGCTTAGTGCAGCCTGGAGGGTCCCGGAAACTCTCCTGTGCAGCCT
CTGGATTCACTTTCAGTAGCTTTGCAATGCACTGGGTTCGTCAGGCTCCAGACAAGGGGCTGGAGTGGGTCGCA
TTCATTAGTAGTGGCAGTAATACCATCTACTATGCAGACACAGTGAAGGGCCGATTCACCGTCTCCAGAGACA
ATCCCAAGAACACCCTGTTCCTGCAAATGACCAGTCTAAGGTCTGAGGACACGGCCATGTATTACTGTGCAAG
TAATTCGTACTATGGTATGGACTACTGGGGTCAAGGAACCTCAGTCACCGTCTCCTCAGCTACAACAACAGCCC
CATCTGTCTATCCCTTGGTCCCTGGCTGCAGTGACACATCTGGATCCTCGGTGACACTGGGATGCCTTGTCAAA
GGCTACTTCCCTGAGCCGGTAACTGTAAAATGGAACTATGGAGCCCTGTCCAGCGGTGTGCGCACAGTCTCAT
CTGTCCTGCAGTCTGGGTTCTATTCCCTCAGCAGCTTGGTGACTGTACCCTCCAGCACCTGGCCCAGCCAGACT
GTCATCTGCAACGTAGCCCACCCAGCCAGCAAGACTGAGTTGATCAAGAGAATCGAGCCTAGAATACCCAAGC
CCAGTACCCCCCCAGGTTCTTCATGCCCACCTGGTAACATCTTGGGTGGACCATCCGTCTTCATCTTCCCCCCAA
AGCCCAAGGATGCACTCATGATCTCCCTAACCCCCAAGGTTACGTGTGTGGTGGTGGATGTGAGCGAGGATGA
CCCAGATGTCCATGTCAGCTGGTTTGTGGACAACAAAGAAGTACACACAGCCTGGACACAGCCCCGTGAAGCT
CAGTACAACAGTACCTTCCGAGTGGTCAGTGCCCTCCCCATCCAGCACCAGGACTGGATGAGGGGCAAGGAGT
TCAAATGCAAGGTCAACAACAAAGCCCTCCCAGCCCCCATCGAGAGAACCATCTCAAAACCCAAAGGAAGAG
CCCAGACACCTCAAGTATACACCATACCCCCACCTCGTGAACAAATGTCCAAGAAGAAGGTTAGTCTGACCTG
CCTGGTCACCAACTTCTTCTCTGAAGCCATCAGTGTGGAGTGGGAAAGGAACGGAGAACTGGAGCAGGATTAC
AAGAACACTCCACCCATCCTGGACTCAGATGGGACCTACTTCCTCTACAGCAAGCTCACTGTGGATACAGACA
GTTGGTTGCAAGGAGAAATTTTTACCTGCTCCGTGGTGCATGAGGCTCTCCATAACCACCACACACAGAAGAA
CCTGTCTCGCTCCCCTGGTAAA
SEQ ID NO: 37 is a nucleic acid sequence encoding the LC of 4D4: 
GACATCCAGATGACTCAGTCTCCAGCCTCCCTATCTGCATCTGTGGGAGAAACTGTCACCATCACATGTCGAAC
AAGTGGGAATATTCGCAATTCATTAGCATGGTATCGACAGAAACAGGGAAAATCCCCTCAGCTCCTGGTCTTT
AATGGCAAAACTTTAGCAGATGGTGTGCCATCAAGGTTCAGTGGCAGTGGATCAGGAACACAATATTCTCTCA
AGATCAACAGCCTGCAGCCTGAAGATTTAGGGACTTATTACTGTCAACATTTTTGGAATATTCCATTCACGTTC
GGCTCGGGGACAAAGTTGGAAATAAAACGGGCTGATGCTGCACCAACTGTATCCATCTTCCCACCATCCAGTG
AGCAGTTAACATCTGGAGGTGCCTCAGTCGTGTGCTTCTTGAACAACTTCTACCCCAAAGACATCAATGTCAAG
TGGAAGATTGATGGCAGTGAACGACAAAATGGCGTCCTGAACAGTTGGACTGATCAGGACAGCAAAGACAGC
 
ACCTACAGCATGAGCAGCACCCTCACGTTGACCAAGGACGAGTATGAACGACATAACAGCTATACCTGTGAGG
CCACTCACAAGACATCAACTTCACCCATTGTCAAGAGCTTCAACAGGAATGAGTGTTAG
SEQ ID NO: 38 is a nucleic acid sequence encoding the HC of 4D4:

CAGGTTCAGCTGCAGCAGTCTGGAGCTGAGCTGATGAAGCCTGGGGCCTCAGTGAAGATATCCTGCAAGGCTC
CTGGCAACACATTCAGTAGCAGGTGGATAGAGTGGGTAAAGGAGAGGCCTGGACATGGCCTTGAGTGGATTG
GAGAGATTTTTCCTGGAAATGGTAATACTAACTACAATGAGAAGTTCAAGGGCAAGGCCACATTCACTGCAGA
TACATCCTCCAACACAGCCTATATACAATTCAGCAGCCTGGCATCTGAGGACTCTGGCGTCTATTACTGTGTAT
CTGAAGACTATTGGGGCCAAGGTACCCTGGTCACCGTCTCCTCAGCCAAGACCACCGCCCCCAGCGTGTACCC
TCTGGCTCCTGTGTGTGGCGATACCACCGGCAGCTCTGTGACCCTGGGCTGCCTCGTGAAGGGATACTTCCCCG
AGCCAGTGACCCTGACCTGGAACAGCGGCTCTCTGAGCAGCGGAGTGCACACCTTTCCAGCCGTGCTGCAGAG
CGACCTGTACACCCTGAGCAGCAGCGTGACCGTGACCTCCAGCACATGGCCCAGCCAGAGCATCACCTGTAAC
GTGGCCCACCCTGCCAGCAGCACCAAGGTGGACAAGAAGATCGAGCCCAGAGGCCCCACCATCAAGCCTTGC
CCCCCTTGCAAATGCCCTGCCCCCAATCTGCTGGGCGGACCCTCCGTGTTTATCTTCCCACCCAAGATCAAGGA
CGTGCTGATGATCAGCCTGAGCCCCATCGTGACCTGCGTGGTGGTGGACGTGTCCGAGGACGACCCCGATGTG
CAGATCAGTTGGTTCGTGAACAACGTGGAAGTGCACACAGCCCAGACCCAGACACACAGAGAGGACTACAAC
AGCACCCTGAGGGTGGTGTCCGCCCTGCCTATCCAGCACCAGGATTGGATGAGCGGCAAAGAATTCAAGTGCA
AAGTGAACAACAAGGACCTGCCAGCCCCCATCGAGCGGACCATCTCTAAGCCTAAGGGCAGCGTGCGGGCTCC
CCAGGTGTACGTGCTGCCTCCTCCAGAGGAAGAGATGACCAAGAAACAAGTGACACTGACATGCATGGTCACC
GACTTCATGCCCGAGGACATCTACGTGGAATGGACCAACAACGGCAAGACCGAGCTGAACTACAAGAATACC
GAGCCCGTGCTGGACAGCGACGGCAGCTACTTCATGTACAGCAAGCTGCGGGTGGAAAAGAAAAACTGGGTG
GAACGGAACAGCTACAGCTGCAGCGTGGTGCACGAGGGCCTGCACAATCACCACACCACCAAGAGCTTCAGC
CGGACCCCCGGCAAATGA

In some embodiments, sequences belonging to the heavy and light chain of an antibody of the present invention are encoded on one or two nucleic acid molecules.

### Host cell

In a third aspect, the present invention provides a host cell comprising the nucleic acid of the present invention.

In some embodiments, the host cell is used in a method for producing the binding protein of the present invention comprising culturing the host cell in conditions suitable for protein expression and then isolating the binding protein.

In some embodiments, the host cell is an immune cell (e.g., a T cell or natural killer cell) and the binding protein (e.g., chimeric antigen receptor or chimeric engulfment receptor) is expressed on the surface of the cell.

### Pharmaceutical composition

In a fourth aspect, the present invention provides a pharmaceutical composition comprising the binding protein, nucleic acid or host cell of the present invention and a pharmaceutically acceptable carrier or diluent.

As used herein, "*pharmaceutically acceptable carrier*" or "*pharmaceutically acceptable diluent*" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone.

A pharmaceutical composition as described herein may also contain other substances. These substances include, but are not limited to, cryoprotectants, lyoprotectants, surfactants, bulking agents, anti-oxidants, and stabilizing agents. In some embodiments, the pharmaceutical composition may be lyophilized.

The term "*cryoprotectant*" as used herein, includes agents which provide stability to the active ingredient against freezing-induced stresses, by being preferentially excluded from the protein's surface. Cryoprotectants may also offer protection during primary and secondary drying and long-term product storage. Non-limiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In one embodiment, a lyoprotectant is added to a pharmaceutical composition described herein. The term "*lyoprotectant*" as used herein, includes agents that provide stability to the active ingredient during the freeze-drying or dehydration process (primary and secondary freeze- drying cycles), by providing an amorphous glassy matrix and by binding with the active ingredient's surface through hydrogen bonding, replacing the water molecules that are removed during the drying process. This helps to minimize product degradation during the lyophilization cycle, and improve the long-term product stability. Non-limiting examples of lyoprotectants include sugars, such as sucrose or trehalose; an amino acid, such as monosodium glutamate, non-crystalline glycine or histidine; a methylamine, such as betaine; a lyotropic salt, such as magnesium sulfate; a polyol, such as trihydric or higher sugar alcohols, e.g., glycerin, erythritol, glycerol, arabitol, xylitol, sorbitol, and mannitol; propylene glycol; polyethylene glycol; pluronics; and combinations thereof. The amount of lyoprotectant added to a pharmaceutical composition is generally an amount that does not lead to an unacceptable amount of degradation of the strain when the pharmaceutical composition is lyophilized.

In some embodiments, a bulking agent is included in the pharmaceutical composition. The term "*bulking agent*" as used herein, includes agents that provide the structure of the freeze- dried product without interacting directly with the pharmaceutical product. In addition to providing a pharmaceutically elegant cake, bulking agents may also impart useful qualities in regard to modifying the collapse temperature, providing freeze-thaw protection, and enhancing the strain stability over long-term storage. Non-limiting examples of bulking agents include mannitol, glycine, lactose, and sucrose. Bulking agents may be crystalline (such as glycine, mannitol, or sodium chloride) or amorphous (such as dextran, hydroxyethyl starch) and are generally used in formulations in an amount from 0.5% to 10%.

Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the desired characteristics of the pharmaceutical composition. As used herein, "pharmaceutically acceptable carrier" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone.

The pharmaceutical composition may be prepared for oral, sublingual, buccal, intravenous, intramuscular, subcutaneous, intraperitoneal, conjunctival, rectal, transdermal, intrathecal, topical and/or inhalation-mediated administration. In a preferred embodiment, the pharmaceutical composition may be a solution which is suitable for intravenous, intramuscular, conjunctival, transdermal, intraperitoneal and/or subcutaneous administration. In an alternative embodiment, the pharmaceutical composition may be a gel or solution which is suitable for intrathecal administration.

The pharmaceutical composition may further comprise common excipients and carriers which are known in the state of the art. For solid pharmaceutical compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For solution for injection, the pharmaceutical composition may further comprise cryoprotectants, lyoprotectants, surfactants, bulking agents, anti-oxidants, stabilizing agents and pharmaceutically acceptable carriers. For aerosol administration, the pharmaceutical compositions are generally supplied in finely divided form along with a surfactant and propellant. The surfactant must, of course, be nontoxic, and is generally soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included, as desired, as with, e.g., lecithin for intranasal delivery. For suppositories, traditional binders and carriers may include, for example, polyalkalene glycols or triglycerides.

### Medical uses

In a fifth aspect, the present invention provides the binding protein, nucleic acid, host cell or pharmaceutical composition of the present invention for use as a medicament. In a sixth aspect, the present invention provides the binding protein, nucleic acid, host cell or pharmaceutical composition of the present invention for use in the treatment and/or prevention of transthyretin amyloidosis (ATTR).

In a particular embodiment, the binding proteins of the invention, which are capable of binding to and clearing or removing isoD-modified TTR in biological fluids and tissues, are useful for the prevention and/or treatment of conditions associated with the formation of TTR-containing aggregates and/or amyloids.

It is believed that the binding proteins of the present invention induce the clearance of aggregated TTR, oligomeric TTR, or monomeric TTR. Clearance is achieved by, for example, targeting the aggregated, oligomeric or monomeric TTR for phagocytosis.

The binding protein, nucleic acid, host cell or pharmaceutical composition of the present invention can be used in a method for treating and/or preventing ATTR, wherein a therapeutically effective amount of binding protein is administered to a subject in need thereof. Patients amenable to treatment include individuals at risk of ATTR but not showing symptoms, as well as patients presently showing symptoms. Some patients can be treated during the prodromal stage of ATTR.

In some embodiments, the ATTR is hereditary ATTR or wild-type ATTR. Examples of hereditary ATTR include familial amyloid cardiomyopathy (FAC), familial amyloid polyneuropathy (FAP), and central nervous system selective amyloidosis (CNSA). Examples of wild-type ATTR include senile systemic amyloidosis (SSA) and senile cardiac amyloidosis (SCA).

In some embodiments, the ATTR is Portuguese-type amyloidosis. Portuguese-type amyloidosis (transthyretin amyloidosis, ATTR V30M) is the most common form of systemic hereditary amyloidosis, inherited in autosomal dominant mode. The disease, also called familial amyloid polyneuropathy type I (FAP-I), is caused by a mutant transthyretin (TTR) protein, which is synthesized by the liver. A single amino acid substitution of methionine for valine at position 30 of the TTR molecule (TTR V30M) was found in Portuguese patients (see Lobato, 2003. J Nephrol. 16(3):438-42).

An exemplary dose range for binding proteins can be from about 0.1-20, or 0.5-5 mg per kg body weight (e.g., 0.5, 1, 2, 3, 4 or 5 mg/kg body weight) or 10-1500 mg as a fixed dosage. The dosage depends on the condition of the patient and response to prior treatme 1-42nt, if any, whether the treatment is prophylactic or therapeutic and whether the disorder is acute or chronic, among other factors.

Compositions of the invention can be administered in such doses daily, on alternative days, weekly, fortnightly, monthly, quarterly, or according to any other schedule determined by empirical analysis. An exemplary treatment entails administration in multiple doses over a prolonged period, for example, of at least six months. Additional exemplary treatment regimens entail administration once per every two weeks or once a month or once every 3 to 6 months.

Compositions can be administered via a peripheral route. Routes of administration include topical, intravenous, oral, subcutaneous, intraarterial, intracranial, intrathecal, intraperitoneal, intranasal or intramuscular. Routes for administration of antibodies can be intravenous or subcutaneous. Intravenous administration can be, for example, by infusion over a period such as 30-90 min. This type of injection is most typically performed in the arm or leg muscles. In some embodiments, agents (e.g., binding proteins, nucleic acids, host cells or pharmaceutical compositions) are injected directly into a particular tissue where deposits have accumulated.

The binding protein, nucleic acid, host cell or pharmaceutical composition of the invention can be administered in combination with another agent effective in treatment or prophylaxis of the disease being treated. Such agents can include siRNA to inhibit expression of TTR or Vyndaqel, a stabilizer of TTR in tetramer formation.

The efficacy of a treatment can be monitored by determining the levels of TTR deposits in a subject by positron emission tomography (PET) before and after administration of the herein disclosed binding protein, nucleic acid, host cell or pharmaceutical composition.

### Diagnosis

IsoD-modified TTR has been detected in extracted human ATTR-V30M fibrils. Without being bound by a particular theory, this indicates that isoD in TTR is a suitable biomarker for the diagnosis and prognosis of ATTR.

In a seventh aspect, the present invention provides an *in vitro* diagnostic and/or prognostic method for detecting the presence of isoD-modified TTR in an isolated sample. In some embodiments, the isolated sample is derived from a patient suspected of suffering from ATTR. In some embodiments, the method comprises:
(i) contacting the isolated sample with a binding protein of the present invention; and
(ii) determining whether isoD-modified TTR is present in the isolated sample.

In some embodiments, the method further comprises determining the ratio of isoD-modified TTR to total TTR. Total TTR can be measured by, for example, using an antibody that specifically binds to TTR without distinguishing between the different forms of TTR. An exemplary antibody that can detect total TTR is "*TTR, Rabbit, polyclonal*" sold by BIOZOL (Biozol, Article Number: CSB-PA041819).

In an eighth aspect, the present invention provides the binding protein of the present invention for use in a method of diagnosis and/or prognosis *in vivo* of ATTR. In some embodiments, the method of diagnosis and/or prognosis comprises administering the binding protein to the individual. In some embodiments, the binding protein is labeled with a detectable moiety (e.g., a radiolabel) and the method allows for the localization of TTR fibrils.

In a preferred embodiment, the binding protein is derivatized in any manner which has been previously discussed. For example, the antibody may be fused to hydrogen peroxidase and used in an ELISA to diagnose and/or prognose ATTR.

Sandwich immunoassays are common in the art. There are plenty of articles available. For example: Cox KL, Devanarayan V, Kriauciunas A, et al. Immunoassay Methods. 2012 May 1 [Updated 2014 Dec 24]. In: Sittampalam GS, Coussens NP, Brimacombe K, et al., editors. Assay Guidance Manual [Internet]. Bethesda (MD): Eli Lilly & Company and the National Center for Advancing Translational Sciences; 2004. Available from: https://www.ncbi.nlm.nih.gov/books/NBK92434/. In a preferred embodiment, the sandwich immunoassay is a direct or indirect ELISA.

### Compositions and methods for producing binding proteins

In a ninth aspect, the present invention provides a method for producing a binding protein (e.g., a binding protein of the present invention) comprising immunizing an animal (e.g., a mouse) with a polypeptide comprising SEQ ID NO: 3.

In a tenth aspect, the present invention provides a method for selecting a binding protein (e.g., a binding protein of the present invention) comprising contacting a population of binding proteins with a polypeptide comprising SEQ ID NO: 3.

In an eleventh aspect, the present invention provides a polypeptide (e.g., an isolated polypeptide) comprising SEQ ID NO: 3. In some embodiments, the isolated polypeptide comprising SEQ ID NO: 3 may be used to produce and/or select a binding protein (e.g., a binding protein of the present invention). In some embodiments, the present invention provides a container comprising a polypeptide (e.g., an isolated polypeptide) comprising SEQ ID NO: 3.

### Items of the invention

The present invention also comprises the following items which may be combined with any embodiment or aspect disclosed in the present application:
[1] A binding protein that specifically binds to an isoaspartate-modified transthyretin.
[2] The binding protein of item [1], wherein the isoaspartate-modified transthyretin comprises SEQ ID NO: 3 and/or the binding protein specifically binds to an epitope comprising an isoaspartate (preferably L-isoaspaitate) residue.
[3] The binding protein of item [1] or [2], wherein the K_{D} of the interaction between the binding protein and SEQ ID NO: 5 is at least 10 times less than the K_{D} of the interaction between the binding protein and SEQ ID NO: 6, wherein, optionally, the K_{D} of the interaction between the binding protein and SEQ ID NO: 5 is at least 200 times less than the K_{D} of the interaction between the binding protein and SEQ ID NO: 6.
[4] The binding protein of any one of items [1]-[3], wherein the K_{D} of the interaction between the binding protein and a polypeptide (e.g., a TTR) comprising SEQ ID NO: 3 is at least 10 times less than the K_{D} of the interaction between the binding protein and a polypeptide (e.g., a TTR) comprising SEQ ID NO: 2, wherein, optionally, the K_{D} of the interaction between the binding protein and a polypeptide (e.g., a TTR) comprising SEQ ID NO: 3 is at least 200 times less than the K_{D} of the interaction between the binding protein and a polypeptide (e.g., a TTR) comprising SEQ ID NO: 2.
[5] The binding protein of item [4], wherein the K_{D} of the interaction between the binding protein and SEQ ID NO: 3 is at least 10 times less than the K_{D} of the interaction between the binding protein and SEQ ID NO: 2, wherein, optionally, the K_{D} of the interaction between the binding protein and SEQ ID NO: 3 is at least 200 times less than the K_{D} of the interaction between the binding protein and SEQ ID NO: 2.
[6] The binding protein of any one of items [1]-[5], wherein the K_{D} of the interaction of the binding protein and a polypeptide (e.g., a TTR) comprising SEQ ID NO: 3 at 25 °C is less than 200 nM.
[7] The binding protein of item [6], wherein the K_{D} of the interaction of the binding protein and SEQ ID NO: 3 at 25 °C is less than 200 nM.
[8] The binding protein of any one of items [1]-[7], wherein the K_{D} of the interaction of the binding protein and SEQ ID NO: 5 at 25 °C is less than 200 nM.
[9] The binding protein of any one of items [1]-[8], wherein the binding protein comprises:
   (a) a heavy chain variable region (VH), wherein said VH comprises a HCDR3 polypeptide selected from:
      (i) NSYYGMDY (SEQ ID NO: 16) or a sequence that has at least 70% (preferably at least 80%) identity thereto; and
      (ii) EDY (SEQ ID NO: 26) or a sequence that has at least 65% identity thereto; and/or
   (b) a light chain variable region (VL), wherein said VL comprises a LCDR3 polypeptide selected from:
      (i) HQYLSSRT (SEQ ID NO: 13) or a sequence that has at least 70% (preferably at least 80%) identity thereto; and
      (ii) QHFWNIPFT (SEQ ID NO: 23) or a sequence that has at least 70% (preferably at least 80%) identity thereto.
[10] The binding protein of any one of items [1]-[9], wherein the binding protein comprises a VL and a VH, wherein said VL comprises LCDR1, LCDR2 and LCDR3 polypeptides and VH comprises HCDR1, HCDR2 and HCDR3 polypeptides which are selected from the group consisting of:
   (a) LCDR1 is KSSQSVLYSSNQKNYLA (SEQ ID NO: 11) or a sequence that has at least 70% (preferably at least 90%) identity thereto, LCDR2 is WASTRES (SEQ ID NO: 12) or a sequence that has at least 70% (preferably at least 80%) identity thereto, LCDR3 is HQYLSSRT (SEQ ID NO: 13) or a sequence that has at least 70% (preferably at least 80%) identity thereto, HCDR1 is GFTFSSFAMH (SEQ ID NO: 14) or a sequence that has at least 70% (preferably at least 90%) identity thereto, HCDR2 is FISSGSNTIYYADTVKG (SEQ ID NO: 15) or a sequence that has at least 70% (preferably at least 90%) identity thereto, and HCDR3 is NSYYGMDY (SEQ ID NO: 16) or a sequence that has at least 70% (preferably at least 80%) identity thereto; and
   (b) LCDR1 is RTSGNIRNSLA (SEQ ID NO: 21) or a sequence that has at least 70% (preferably at least 90%) identity thereto, LCDR2 is NGKTLAD (SEQ ID NO: 22) or a sequence that has at least 70% (preferably at least 80%) identity thereto, LCDR3 is QHFWNIPFT (SEQ ID NO: 23) or a sequence that has at least 70% (preferably at least 80%) identity thereto, HCDR1 is GNTFSSRWIE (SEQ ID NO: 24) or a sequence that has at least 70% (preferably at least 90%) identity thereto, HCDR2 is EIFPGNGNTNYNEKFKG (SEQ ID NO: 25) or a sequence that has at least 70% (preferably at least 90%) identity thereto, and HCDR3 is EDY (SEQ ID NO: 26) or a sequence that has at least 65% identity thereto.
[11] The binding protein of any one of items [1]-[10], wherein the binding protein comprises a VL and a VH, wherein said VL comprises LCDR1, LCDR2 and LCDR3 polypeptides and VH comprises HCDR1, HCDR2 and HCDR3 polypeptides which are selected from the group consisting of:
   (a) LCDR1 is KSSQSVLYSSNQKNYLA (SEQ ID NO: 11), LCDR2 is WASTRES (SEQ ID NO: 12), LCDR3 is HQYLSSRT (SEQ ID NO: 13), HCDR1 is GFTFSSFAMH (SEQ ID NO: 14), HCDR2 is FISSGSNTIYYADTVKG (SEQ ID NO: 15), and HCDR3 is NSYYGMDY (SEQ ID NO: 16); and
   (b) LCDR1 is RTSGNIRNSLA (SEQ ID NO: 21), LCDR2 is NGKTLAD (SEQ ID NO: 22), LCDR3 is QHFWNIPFT (SEQ ID NO: 23), HCDR1 is GNTFSSRWIE (SEQ ID NO: 24), HCDR2 is EIFPGNGNTNYNEKFKG (SEQ ID NO: 25), and HCDR3 is EDY (SEQ ID NO: 26).
[12] The binding protein of any one of items [1]-[11], wherein the binding protein comprises a VL and a VH, wherein the VL and VH are polypeptides selected from the group consisting of: (a) VL of SEQ ID NO: 17 or a sequence that has at least 50% identity thereto, and VH of SEQ ID NO: 18 or a sequence that has at least 50% identity thereto; (b) VL of SEQ ID NO: 27 or a sequence that has at least 50% identity thereto, and VH of SEQ ID NO: 28 or a sequence that has at least 50% identity thereto; (c) VL of SEQ ID NO: 17 or a sequence that has at least 50% identity thereto, and VH of SEQ ID NO: 28 or a sequence that has at least 50% identity thereto; and (d) VL of SEQ ID NO: 27 or a sequence that has at least 50% identity thereto, and VH of SEQ ID NO: 18 or a sequence that has at least 50% identity thereto.
[13] The binding protein of any one of items [1]-[12], wherein the binding protein is an antibody.
[14] A nucleic acid comprising a sequence that encodes the binding protein of any one of items [1]-[13].
[15] A host cell comprising the nucleic acid of item [14].
[16] A pharmaceutical composition comprising the binding protein of any one of items [1]-[13], the nucleic acid of item [14] or the host cell of item [15], and a pharmaceutically acceptable carrier or diluent.
[17] The binding protein of any one of items [1]-[13], the nucleic acid of item [14], the host cell of item [15], or the pharmaceutical composition of item [16] for use as a medicament.
[18] The binding protein of any one of items [1]-[13], the nucleic acid of item [14], the host cell of item [15], or the pharmaceutical composition of item [16] for use in the treatment and/or prevention of transthyretin amyloidosis.
[19] An *in vitro* diagnostic and/or prognostic method for detecting the presence of isoaspartate-modified transthyretin in an isolated sample, the method comprising:
   (i) contacting the isolated sample with the binding protein according to any one of items [1]-[20]; and
   (ii) determining whether isoaspartate-modified transthyretin is present in the isolated sample.
[21] The binding protein of any one of items [1]-[13] for use in a method of diagnosis and/or prognosis *in vivo* of transthyretin amyloidosis.

### Examples

### Example 1: Preparation and screening of monoclonal antibodies directed against IsoAsp38 TTR

The aim of this study was to generate monoclonal antibodies that react with isoAsp38-TTR and shorter peptides thereof containing isoaspartate at position 38 (e.g., SEQ ID NO: 3), but not with the same molecules possessing an aspartate at position 38 (e.g., SEQ ID NO: 2).

A peptide containing amino acids 35-43 of human TTR, with an isoaspartate residue at position 38 instead of aspartate and an additional artificial C-terminal amino acid for ease of coupling (i.e., KAAXDTWEPC, wherein X is L-isoAsp) (SEQ ID NO: 4), was synthesized by Peptide&Elephants (Berlin, Germany). The peptide was covalently linked to keyhole limpet hemocyanin via the C-terminal cysteine residue. For the generation of monoclonal antibodies, 8-week-old female BALB/c mice were used. Mice were immunized subcutaneously with a 1:1 emulsification of KLH-conjugated TTR peptide and Freund's complete adjuvant (VWR), followed by boosts with another 95 µg in incomplete Freund's adjuvant 44 and 73 days later and a final immunogen boost every day starting 4 days prior to fusion. Antibody titer was confirmed by an enzyme-linked immunosorbent assay (ELISA). Homogenized spleen cells were fused to SP2/0 myeloma cells using the PEG method (Köhler & Milstein, 1975. Nature. 256(5517):495-7). Cells were immediately plated on prepared feeder cell plates in HAT medium and screened after 10 days. Hybridomas were selected upon specific binding to isoD38-TTR(35-43)-PEG-Biotin (SEQ ID NO: 5), but not to TTR(35-43)-PEG-Biotin (SEQ ID NO: 6), using an indirect ELISA and surface plasmon resonance (SPR). Stable antibody-producing hybridomas have been selected and subsequently cloned by limited dilution and an additional single-cell-separation to ensure the monoclonality of the hybridomas.

Hybridoma cell clones were expanded and cultured in T175 flasks. The supernatant was collected and sterile filtered (0.22 µm, TPP) prior to purification. For affinity purification of the mAbs, the respective supernatant was mixed with binding buffer (40 mM Na₂HPO₄, 150 mM NaCl, pH 7.0) at an equal volume and subjected to a pre-equilibrated Protein G Sepharose Fast Flow column (GE Healthcare Life Sciences). Elution of bound antibodies was done by using 40 mM Na₂HPO₄ and 2 M KSCN at pH 7.0. Collected fractions were dialyzed against PBS (138 mM NaCl, 8 mM Na₂HPO₄, 1.5 mM KH₂PO₄, 3 mM KCI, pH 7.0) at 4 °C overnight.

### Example 2: Investigation of antibody specificity by Dot Blot analysis

A Dot Blot analysis was performed to obtain a general idea about the specificity and cross-reactivity of the antibodies obtained in Example 1.

### Methods

0.5 µg of recombinantly expressed TTR proteins (TTRwt (SEQ ID NO: 1), TTR-V30M (SEQ ID NO: 7), or TTR-V122I (SEQ ID NO: 8)), ATTR fibrils or 0.5 µg of either freshly dissolved Aβ(1-42) (SEQ ID NO: 9) or aggregated Aβ(1-42) with confirmed isoAsp-content (SEQ ID NO: 10) were spotted on nitrocellulose membranes. Membranes were blocked for 1 hour in blocking solution (5 % (w/v) milk powder in TBS + 0.05 % (v/v) Tween-20 (Carl Roth)). Antibodies 2F2, 4D4 or polyclonal total-TTR antibody (Biozol, article nr. CSB-PA041819) were diluted to 1 µg/mL in blocking solution and incubated overnight at 4 °C. Alkaline phosphatase-conjugated, anti-mouse or rabbit IgG antibodies (abeam) in combination with chromogenic substrates BCIP and NBT (Carl Roth) were subsequently used for detection.
SEQ ID NO: 7 is TTR-V30M and has the following sequence: 
GPTGTGESKCPLMVKVLDAVRGSPAINVAMHVFRKAADDTWEPFASGKTSESGELHGLTTEE
EFVEGIYKVEIDTKSYWKALGISPFHEHAEVVFTANDSGPRRYTIAALLSPYSYSTTAVVTNPK
E
SEQ ID NO: 8 is TTR-V122I and has the following sequence: 
GPTGTGESKCPLMVKVLDAVRGSPAINVAVHVFRKAADDTWEPFASGKTSESGELHGLTTEE
EFVEGIYKVEIDTKSYWKALGISPFHEHAEVVFTANDSGPRRYTIAALLSPYSYSTTAVITNPK
E
SEQ ID NO: 9 is Aβ(1-42) and has the following sequence:
   DAEFRHDSGYEVHHQKLVFFAEDVGSNKGAIIGLMVGGVVIA
SEQ ID NO: 10 is isoD7-Aβ(1-42) and has the following sequence:
   DAEFRHXSGYEVHHQKLVFFAEDVGSNKGAIIGLMVGGVVIA,
   wherein X is L-isoAp.

### Results

As shown in FIG. 1, recombinantly expressed, monomeric proteins of TTRwt, TTR-V30M and TTR-V122I (Lane 1-3) were not identified by 2F2 and 4D4 antibody, indicating absence of isoaspartate residues at position 38. However, 2F2 and 4D4 recognized aggregated, one-year-old samples of the recombinant TTR-derived fibrils (Lane 4 - 6). Furthermore, isoD38 TTR species were observed in extracted fibrils of human cardiac tissue of patients with pathological ATTR-V30M, detected by 2F2 and 4D4 antibody (Lane 7). In contrast, the total-TTR pAb did not discriminate between recombinant monomer TTR variants and aggregated, aged TTR fibrils either produced *in vitro* or extracted from human tissue (Column 3). Both TTR mAbs showed no reactivity against other isoaspartate-containing amyloid fibrils (isoD7-Aβ) or their monomeric peptides (Lane 8 and 9).

### Example 3: K_{D} value determination of immobilized 2F2 or 4D4 with TTR(35-43) or isoD38-TTR(35-43) by surface plasmon resonance (SPR) analysis

### Methods

Antibody binding kinetics to TTR(35-43)-PEG-Biotin (SEQ ID NO: 5) and isoD38-TTR(35-43)-PEG-Biotin (SEQ ID NO: 6) were analyzed by using SPR on a BIAcore 3000 device at 25 °C. For this purpose, the surface of a CM5 sensor chip (Cytiva) was modified with a goat anti-mouse antibody using NHS/EDC chemistry. To immobilize the anti-mouse IgG, the carboxymethylated dextran surface of the sensor chip was activated by mixing 0.1 M N-hydroxysuccinimide (NHS) with 0.4 M N-ethyl-N'-(dimethylaminopropyl) carbodiimide hydrochloride (EDC) 1:1. EDC/NHS was applied to the sensor chip with a flow rate of 20 µL/min for 7 minutes. Goat anti-mouse IgG was diluted to 50 µg/mL in 10 mM sodium acetate, pH 5.5, and injected for 2 x 4 minutes contact time with a flow rate of 30 µL/min. Deactivation was done by applying 1 M ethanolamine pH 8.5 with a flow rate of 10 µL/min for 2 x 7 minutes contact time. About 20000 RU of the goat anti-mouse antibody were immobilized onto the chip surface. The antibodies 2F2 and 4D4 were captured using antibody dilutions of 25 µg/mL in HBS-EP buffer and a flow rate of 2 µL/min to achieve a level of about 2000 RU for both antibodies on the chip surface. For 4D4 antibody, multicycle kinetic analysis was carried out by applying 10 nM to 100 µM of isoD38-TTR(35-43)-PEG-Biotin (SEQ ID NO: 5) and 1 µM to 200 µM of TTR(35-43)-PEG-Biotin (SEQ ID NO: 6), respectively. For 2F2 antibody, five consecutive injections of 2 nM to 512 nM of isoD38-TTR(35-43)-PEG-Biotin (SEQ ID NO: 5) and 512 nM to 131 µM TTR(35-43)-PEG-Biotin (SEQ ID NO: 6) were applied.

### Results

The dissociation constants for the binding of 2F2 and 4D4 to either isoD38-TTR(35-43)-PEG-Biotin (SEQ ID NO: 5) or TTR(35-43)-PEG-Biotin (SEQ ID NO: 6) are shown in TABLE 1. Both TTR mAbs showed high selectivity for the isoaspartate-containing TTR(35-43)-peptide (SEQ ID NO: 5). In comparison, the dissociation constants for the non-modified peptide TTR(35-43) (SEQ ID NO: 6) are 233- (4D4) and 464-fold (2F2) lower.

**Table 1: K_{D} values between isoD38-TTR(35-43)-specific antibodies and TTR(35-43)-peptides**

| **mAb** | **Peptide** | **K_{D} (nM)** |
|---|---|---|
| 2F2 | isoD38-TTR(35-43) | 1.55 |
| 4D4 | | 124 |
| 2F2 | TTR(35-43) | 719 |
| 4D4 | | 29,000 |

### Example 4: Engulfment and degradation of TTR fibrils by phagocytosis using isoD38-TTR mAbs 2F2 and 4D4

In order to analyze, whether the antibodies 2F2 and 4D4 are capable of inducing Fc-mediated phagocytosis of TTR fibrils, we established a cellular phagocytosis assay using macrophage-like THP-1 cells *in vitro.* THP-1 cells are commonly used to assess the ability of a monoclonal antibody to induce Fc-mediated phagocytosis (see, for example, Gogesch et al., 2021. Int J Mol Sci. 22(16): 8947, section 2.4.2).

### Methods

A sample containing heart extracted human ATTR-V30M fibrils or 1 mg/mL native recombinant TTRwt (SEQ ID NO: 1) was amine-coupled with pHrodo Red dye (Thermo Fisher, see, for example, Miksa et al., 2009. J Immunol Methods. 342(1-2):71-7) at room temperature for 1 hour, applying a protein:dye ratio of 10:1. In the case of native TTRwt, excess pHrodo Red dye was removed by diafiltration using a 5 kDa molecular weight cut-off (Merck). For amyloid fibrils, three centrifugation steps at 17.000 xg for 10 minutes followed by resuspension in PBS removed excess free dye label.

For stimulation, 2 x 10⁵ human monocytes THP-1 cells were cultured in cell culture media (RPMI, 10% low IgG FBS) and differentiated using 200 nM phorbol 12-myristate 13-acetate (PMA, Sigma-Aldrich) for 3 days. After this initial stimulus, PMA-containing media was replaced by fresh RPMI + 10% low IgG FBS for 2 further days.

A 20 µg/mL aliquot of pHrodo-labelled TTR (either monomer TTRwt or extracted human TTR-V30M fibril) was separately pre-incubated with 40 µg/mL antibody in cell culture media at 37 °C for 1 hour. After addition of 2 x 10⁵ PMA-treated THP-1 cells the tissue culture was incubated for 3 hours. Antigen-antibody complexes were removed by washing the cells three times with PBS.

The extent of phagocytosis was monitored by flow cytometry using a FACSCalibur (Becton Dickinson). Forward and side scattering light was used to identify cell populations and to measure size and granularity of the cells. The red fluorescence emitted by the cells was recorded at 585 nm after excitation at 560 nm. Remaining cells were transferred to glass chamber slides and imaged by bright field and fluorescence microscopy using a Keyence BZ-X800.

### Results

The extracted human ATTR-V30M fibrils or, as a control, recombinant monomeric TTRwt were covalently conjugated to the pH-sensitive dye pHrodo-red. At physiological pH, this dye has minimal fluorescence, whereas in an acidic environment, as in endocytic vesicles, the fluorescence is strongly increased. This allows one to monitor the cellular uptake of pHrodo-labelled proteins. THP-1 cells were added after treatment of the pHrodo-labelled TTR with the two antibodies 2F2 and 4D4 or isotype control, respectively. The cellular uptake was visualized by bright field and fluorescence microscopy (FIG. 2A) and measured by flow cytometry and displayed as mean fluorescence intensity (MFI, FIG. 2B). Low fluorescence was observed with either TTRwt monomer treated with isoD38-TTR-specific antibodies or the isotype controls. In contrast, fluorescence was significantly increased after treatment of ATTR-V30M fibrils with 2F2 or 4D4 antibody, indicating phagocytosis of TTR fibrils mediated by these antibodies.

These data demonstrate that targeting isoD38 allows for the clearance of TTR fibrils via antibody-dependent cellular phagocytosis. The ability of an antibody to reduce TTR amyloid load is a therapeutic goal anticipated to translate into improved patient survival (see Michalon et al., 2021. Nat Commun. 12(1):3142). Thus, the present data makes it credible that the binding proteins disclosed herein would be suitable for treating transthyretin amyloidosis.

## Claims

1. A binding protein that specifically binds to an isoaspartate-modified transthyretin.

2. The binding protein of claim 1, wherein the isoaspartate-modified transthyretin comprises SEQ ID NO: 3 and/or the binding protein specifically binds to an epitope comprising an isoaspartate residue.

3. The binding protein of claim 1 or 2, wherein the K_{D} of the interaction between the binding protein and SEQ ID NO: 5 is at least 10 times less than the K_{D} of the interaction between the binding protein and SEQ ID NO: 6, wherein, optionally, the K_{D} of the interaction between the binding protein and SEQ ID NO: 5 is at least 200 times less than the K_{D} of the interaction between the binding protein and SEQ ID NO: 6.

4. The binding protein of any one of claims 1-3, wherein the K_{D} of the interaction of the binding protein and SEQ ID NO: 5 at 25 °C is less than 200 nM.

5. The binding protein of any one of claims 1-4, wherein the binding protein comprises:
(a) a heavy chain variable region (VH), wherein said VH comprises a HCDR3 polypeptide selected from:
(i) NSYYGMDY (SEQ ID NO: 16) or a sequence that has at least 70% (preferably at least 80%) identity thereto; and
(ii) EDY (SEQ ID NO: 26) or a sequence that has at least 65% identity thereto; and/or
(b) a light chain variable region (VL), wherein said VL comprises a LCDR3 polypeptide selected from:
(i) HQYLSSRT (SEQ ID NO: 13) or a sequence that has at least 70% (preferably at least 80%) identity thereto; and
(ii) QHFWNIPFT (SEQ ID NO: 23) or a sequence that has at least 70% (preferably at least 80%) identity thereto.

6. The binding protein of any one of claims 1-5, wherein the binding protein comprises a VL and a VH, wherein said VL comprises LCDR1, LCDR2 and LCDR3 polypeptides and VH comprises HCDR1, HCDR2 and HCDR3 polypeptides which are selected from the group consisting of:
(a) LCDR1 is KSSQSVLYSSNQKNYLA (SEQ ID NO: 11) or a sequence that has at least 70% (preferably at least 90%) identity thereto, LCDR2 is WASTRES (SEQ ID NO: 12) or a sequence that has at least 70% (preferably at least 80%) identity thereto, LCDR3 is HQYLSSRT (SEQ ID NO: 13) or a sequence that has at least 70% (preferably at least 80%) identity thereto, HCDR1 is GFTFSSFAMH (SEQ ID NO: 14) or a sequence that has at least 70% (preferably at least 90%) identity thereto, HCDR2 is FISSGSNTIYYADTVKG (SEQ ID NO: 15) or a sequence that has at least 70% (preferably at least 90%) identity thereto, and HCDR3 is NSYYGMDY (SEQ ID NO: 16) or a sequence that has at least 70% (preferably at least 80%) identity thereto; and
(b) LCDR1 is RTSGNIRNSLA (SEQ ID NO: 21) or a sequence that has at least 70% (preferably at least 90%) identity thereto, LCDR2 is NGKTLAD (SEQ ID NO: 22) or a sequence that has at least 70% (preferably at least 80%) identity thereto, LCDR3 is QHFWNIPFT (SEQ ID NO: 23) or a sequence that has at least 70% (preferably at least 80%) identity thereto, HCDR1 is GNTFSSRWIE (SEQ ID NO: 24) or a sequence that has at least 70% (preferably at least 90%) identity thereto, HCDR2 is EIFPGNGNTNYNEKFKG (SEQ ID NO: 25) or a sequence that has at least 70% (preferably at least 90%) identity thereto, and HCDR3 is EDY (SEQ ID NO: 26) or a sequence that has at least 65% identity thereto.

7. The binding protein of any one of claims 1-6, wherein the binding protein comprises a VL and a VH, wherein said VL comprises LCDR1, LCDR2 and LCDR3 polypeptides and VH comprises HCDR1, HCDR2 and HCDR3 polypeptides which are selected from the group consisting of:
(a) LCDR1 is KSSQSVLYSSNQKNYLA (SEQ ID NO: 11), LCDR2 is WASTRES (SEQ ID NO: 12), LCDR3 is HQYLSSRT (SEQ ID NO: 13), HCDR1 is GFTFSSFAMH (SEQ ID NO: 14), HCDR2 is FISSGSNTIYYADTVKG (SEQ ID NO: 15), and HCDR3 is NSYYGMDY (SEQ ID NO: 16); and
(b) LCDR1 is RTSGNIRNSLA (SEQ ID NO: 21), LCDR2 is NGKTLAD (SEQ ID NO: 22), LCDR3 is QHFWNIPFT (SEQ ID NO: 23), HCDR1 is GNTFSSRWIE (SEQ ID NO: 24), HCDR2 is EIFPGNGNTNYNEKFKG (SEQ ID NO: 25), and HCDR3 is EDY (SEQ ID NO: 26).

8. The binding protein of any one of claims 1-7, wherein the binding protein comprises a VL and a VH, wherein the VL and VH are polypeptides selected from the group consisting of: (a) VL of SEQ ID NO: 17 or a sequence that has at least 50% identity thereto, and VH of SEQ ID NO: 18 or a sequence that has at least 50% identity thereto; (b) VL of SEQ ID NO: 27 or a sequence that has at least 50% identity thereto, and VH of SEQ ID NO: 28 or a sequence that has at least 50% identity thereto; (c) VL of SEQ ID NO: 17 or a sequence that has at least 50% identity thereto, and VH of SEQ ID NO: 28 or a sequence that has at least 50% identity thereto; and (d) VL of SEQ ID NO: 27 or a sequence that has at least 50% identity thereto, and VH of SEQ ID NO: 18 or a sequence that has at least 50% identity thereto.

9. The binding protein of any one of claims 1-8, wherein the binding protein is an antibody.

10. A nucleic acid comprising a sequence that encodes the binding protein of any one of claims 1-9.

11. A host cell comprising the nucleic acid of claim 10.

12. The binding protein of any one of claims 1-9, the nucleic acid of claim 10, or the host cell of claim 11 for use as a medicament.

13. The binding protein of any one of claims 1-9, the nucleic acid of claim 10, or the host cell of claim 11 for use in the treatment and/or prevention of transthyretin amyloidosis.

14. An *in vitro* diagnostic and/or prognostic method for detecting the presence of isoaspartate-modified transthyretin in an isolated sample, the method comprising:
(i) contacting the isolated sample with the binding protein according to any one of claims 1-9; and
(ii) determining whether isoaspartate-modified transthyretin is present in the isolated sample.

15. The binding protein of any one of claims 1-9 for use in a method of diagnosis and/or prognosis *in vivo* of transthyretin amyloidosis.
